# EUROPÄISCHE PATENTANMELDUNG

(11) **EP 4 095 230 A1**
(43) Veröffentlichungstag der Anmeldung: **30.11.2022**
(21) Anmeldenummer: 22174157.2
(22) Anmeldetag: 18.05.2022
(51) Int. Cl.: C12N 1/18, C12N 1/32, C12P 7/46

(54) **GENTECHNISCH VERÄNDERTE HEFE ZUR BIOTECHNOLOGISCHEN HERSTELLUNG VON BERNSTEINSÄURE AUS GLYCERIN**

(30) Priorität: 26.05.2021 DE 102021113602
(71) Anmelder: Jacobs University Bremen gGmbH, 28759 Bremen (DE)
(72) Erfinder: Nevoigt, Elke, 10439 Berlin (DE)
(74) Vertreter: Weidner Stern Jeschke

(57) **Zusammenfassung**

Die Erfindung betrifft eine gentechnisch veränderte Hefe zur biotechnologischen Herstellung von Bernsteinsäure aus Glycerol, wobei die Hefezelle dahingehend gentechnisch verändert ist, dass
a) der Abbau von Glycerol zu Dihydroxyacetonphosphat über den Glycerol-3-Phosphat-Weg blockiert ist, und
b) die folgenden Gene in den LTR-Insertionslokus YPRCτ3 exprimierbar inseriert sind:
i) *ScMDH3-R* unter Kontrolle des Promotors *P_{JEN1}* und funktional verbunden mit der Terminationssequenz *T_{IDP1,}*
ii) *RofumR* unter Kontrolles des Promotors *P_{HOR7}* und funktional verbunden mit der Terminationssequenz *T_{DIT1},*
iii) *TbFRDg-R* unter Kontrolle des Promotors *P_{FBA1}* und funktional verbunden mit der Terminationssequenz *T_{ADH1},*
iv) *AnDCT02* unter Kontrolle des Promotors *P_{COX7}* und funktional verbunden mit der Terminationssequenz *T_{CYC1},* und

c) die folgenden Gene in den LTR-Insertionslokus YGLCτ3 exprimierbar inseriert sind:
i) *CjFPS1* unter Kontrolle des Promotors *P_{TDH3}* und funktional verbunden mit der Terminationssequenz *T_{RPL15A},*
ii) *Opgdh* unter Kontrolles des Promotors *P_{TEF1}* und funktional verbunden mit der Terminationssequenz *T_{CYC1},* und
iii) *ScDAK1* unter Kontrolle des Promotors *P_{ADH2}* und funktional verbunden mit der Terminationssequenz *T_{TPS1}.*

## Beschreibung

Die Erfindung betrifft eine gentechnisch veränderte Hefe zur biotechnologischen Herstellung von Bernsteinsäure aus Glycerol.

Die Bäckerhefe (*Saccharomyces cerevisiae*) wächst nur schlecht auf Glycerol (Glycerin) als Kohlenstoffquelle. Die in der Forschung und industriell häufig verwendeten Stämme von *S. cerevisiae* benötigen Zugabe von Ergänzungsstoffen wie beispielsweise Aminosäuren und Nukleobasen, um auf Glycerol zu wachsen (Merico et al 2011; Swinnen et al., 2013). Lediglich einzelne Isolate sind in der Lage, Glycerol ohne solche Medienzusätze als Kohlenstoffquelle zu nutzen (Swinnen et al. 2013).

Die Herstellung von C₄-Dicarbonsäuren, zum Beispiel Bernsteinsäure, durch die in der Biotechnologie verbreitet genutzte Bäckerhefe ist gegenüber der Herstellung durch Bakterien vorteilhaft, weil der Prozess beispielsweise bei niedrigem pH erfolgen kann, wodurch auf den Einsatz neutralisierender Verbindungen verzichtet und auch die Aufreinigung des Produktes erleichtert werden kann. Die biotechnologische Herstellung von Bernsteinsäure (Butandisäure) bzw. Salzen davon (Succinate) mittels Hefe ist daher von hohem kommerziellem Interesse (s. z.B. McKinlay et al., 2007).

Die biotechnologische Herstelltung von Bernsteinsäure kann grundsätzlich über drei Stoffwechselwege erfolgen, (i) den oxidativen Zweig des Citratzyklus, (ii) den reduktiven Zweig des Citratzyklus (rTCA) und (iii) den Glyoxylatzyklus, wobei der Weg unter Nutzung des reduktiven Citratzyklus aufgrund der dabei erfolgenden CO₂-Fixierung die höchste Ausbeute verspricht (s. Xiberras et al. 2020). Bernsteinsäurebildung aus Glucose als einziger Kohlenstoffquelle durch *S. cerevisiae* wurde von mehreren Autoren beschrieben (Raab et al., 2010; Otero et al., 2013; Yan et al., 2014). Ein wesentlicher Vorteil bei der Nutzung von Glycerol als einziger oder zusätzlicher Kohlenstoffquelle in der industriellen Biotechnologie besteht darin, dass der Glycerol-Katabolismus im Vergleich zu Glucose pro C-Mol mehr Reduktionsäquivalente liefert. Dies ermöglicht grundsätzlich höhere maximale theoretische Ausbeuten von Stoffwechselprodukten, deren Syntheseweg Reduktionsäquivalente wie beispielsweise NADH oder NADPH erfordert, wie beispielsweise Bernsteinsäure, zumindest wenn diese über den reduktiven Weg gebildet werden.

Es ist im Stand der Technik bekannt, die Bäckerhefe gentechnisch so zu verändern, dass sie in die Lage versetzt ist, Glycerol besser zur Bildung von C₄-Dicarbonsäuren wie beispielsweise Succinat zu nutzen. Aus der WO 2016/008819 A1 ist beispielsweise eine gentechnisch veränderte Hefe mit verbessertem Glycerol-Katabolismus bekannt. Die Hefezelle wurde dabei dahingehend gentechnisch verändert, dass der Abbau von Glycerol über den Glycerol-3-Phosphat-Weg (G3P-Weg) blockiert ist, ein heterologes Glycerolaufnahme-Channelprotein (Aquaglyceroporin) exprimiert wird, und entweder der DHA-Weg etabliert oder verstärkt ist, oder der Glycerinaldehyd-Weg etabliert oder optimiert ist.

Die WO 2018/033176 A1 offenbart eine gentechnisch veränderte Hefezelle mit einem heterologen Glycerolaufnahme-Channelprotein für einen energieunabhängigen Glycerol-Import, einem blockierten G3P-Glycerol-Abbau-Weg, einem Glycerol-Abbau über den DHA-Weg unter Bildung von cytosolischem NADH und einem verbesserten Export von gebildeten C₄-Carbonsäuren durch einen heterologen C₄-Dicarbonsäure Transporter. Darüber hinaus ist die Hefezelle mit einem Stoffwechselweg zur Bildung von 1,3-Propandiol als Redoxsenke für bei der Biomasseproduktion gebildetes NADH ausgestattet.

Xiberras et al. 2020, deren Offenbarung hier durch Inbezugnahme vollständig aufgenommen ist, beschreiben die gentechnische Veränderung von *Saccharomyces cerevisiae* für die Bernsteinsäure-Produktion aus Glycerol und Kohlendioxid durch den (cytosolischen) reduktiven Weg. Die Hefezelle ist gentechnisch so verändert, dass sie in die Lage versetzt ist, Bernsteinsäure aus Glycerol über den DHA-Weg und einen reduktiven Kohlendioxid-fixierenden und redoxneutralen Weg zu Bernsteinsäure abzubauen und über den heterologen Dicarbonsäuretransporter DCT-02 aus *Aspergillus niger* in das Anzuchtsmedium auszuscheiden. Der G3P-Weg ist blockiert und die Zelle ist zur verbesserten Glycerolaufnahme mit dem Gen für den heterologen Glycerolaufnahme-Facilitator (Aquaglyceroporin) Fps1 aus *Cyberlindnera jadinii* ausgestattet. Der G3P-Weg wurde blockiert durch Insertion von Expressionskassetten für Gene des DHA-Weges an dem Genlokus für *GUT1,* das für die Glycerolkinase kodiert, dem ersten Enzym des G3P-Wegs (s. z.B. Klein et al. 2017). Das Hefe-Gen für Dihydroxyacetonkinase (*ScDAK1*) wurde dabei zur Überexpression zweimal ins Genom inseriert, jedoch mit unterschiedlichen Promotoren und Terminatoren (*P_{ACT1}* und *T_{TPS1}* sowie *P_{TDH3}* und T*_{IDP1}*), und darüber hinaus wurde ein Gen für die heterologe NAD-abhängige Glyceroldehydrogenase aus *Ogataea parapolymorpha* (*Opgdh*) einmal unter Kontrolle des Promotors P*_{TEF1}* und mit dem Terminator T*_{CYC1}* inseriert. Darüber hinaus wurde an diesem *GUT1-*Lokus noch die Expressionskassette für den Glycerolaufnahme-Channel Fps1 aus *C. jadinii* (*CjFPS1*) inseriert, mit dem Promotor P*_{PGK1}* und dem Terminator T*_{RPL15A}.* Der gentechnisch veränderte *Saccharomyces*-Stamm wies darüber hinaus noch das in das Genom integrierte CBS-Allel *UBR2_{CBS}* (s. Swinnen et al. 2016) auf, welches das native Allel ersetzt und ein entscheidender Faktor für eine hohe Wachstumsrate des eingesetzten Stammes ist (s. Klein et al. 2016). Zur Produktion von Bernsteinsäure wurden noch Expressionskassetten für Enzymgene des reduktiven Wegs zur Umsetzung von Oxalacetat zu Succinat ("SA-Modul") sowie für den Dicarbonsäuretransporter DCT-02 (*AnDCT-02*) an zwei unterschiedlichen chromosomalen Long-Terminal-Repeat(LTR)-Insertionsstellen (s. Bai Flagfeldt et al. 2009) inseriert. Das "SA-Modul" wurde in den LTR-Insertionslokus YGLCτ3 (s. Bai Flagfeldt et al. 2009, Tabelle 2, Nr. 8) inseriert und umfasst Expressionskassetten für a) eine peroxisomale Hefe-Malatdehydrogenase (*ScMDH3-R*) unter Kontrolle des Promotors T*_{PGK1}* und des Terminators P*_{IDP1},* die durch Entfernen der peroxisomalen Translokationssequenz im Cytosol verbleibt, b) eine heterologe Fumarase aus *Rhizopus oryzae* (*RofumR*) unter Kontrolle des Promotors P*_{TEF1}* und des Terminators T*_{RPL15A}* und c) eine heterologe peroxisomale Fumaratreduktase aus *Trypanosoma brucei* (*TbFRDg-R*) unter Kontrolle des Promotors P*_{TDH3}* und des Terminators T*_{CYC1},* die ebenfalls durch Entfernen des entsprechenden Signalpeptids im Cytosol verbleibt. In den LTR-Insertionslokus YPRCτ3 (s. Bai Flagfeldt et al. 2009, Tabelle 2, Nr. 21) wurde die Expressionskassette für den Dicarbonsäuretransporter (SA-Transporter) aus *Aspergillus niger* (*AnDCT*) unter Kontrolle des Promotors P*_{ENO2}* und des Terminators T*_{DIT1}* inseriert. Xiberras et al. 2020 berichten von einem maximalen Bernsteinsäuretiter von 10,7 g/L und einem Ertrag von 0,22 ± 0,01 g/g Glycerol in Schüttelkolben-Batch-Kultivierung.

Trotz des oben beschriebenen Ansatzes, Hefe gentechnisch für die Umsetzung von Glycerol zu Bernsteinsäure zu optimieren, besteht nach wie vor Bedarf an einem Hefestamm mit verbesserten Eigenschaften zur biotechnologischen Herstellung von Bernsteinsäure.

Aufgabe der vorliegenden Erfindung ist es, eine Hefezelle mit gegenüber dem Stand der Technik verbesserter Umsetzung von Glycerol zu Bernsteinsäure bereitzustellen. Insbesondere soll durch die Erfindung die Herstellung von Bernsteinsäure durch Hefe mit einer verbesserten Produktausbeute und -bildungsrate ermöglicht werden.

Gelöst wird die Aufgabe durch eine gentechnisch veränderte Hefezelle der Gattung *Saccharomyces* zur biotechnologischen Herstellung von Bernsteinsäure aus Glycerol, wobei die Hefezelle dahingehend gentechnisch verändert ist, dass
a) der Abbau von Glycerol zu Dihydroxyacetonphosphat über den Glycerol-3-Phosphat-Weg blockiert ist, und
b) die folgenden Gene in den LTR-Insertionslokus YPRCτ3 exprimierbar inseriert sind:
   i) *ScMDH3-R* unter Kontrolle des Promotors P*_{JEN1}* und funktional verbunden mit der Terminationssequenz T*_{IDP1},*
   ii) *RofumR* unter Kontrolles des Promotors P*_{HOR7}* und funktional verbunden mit der Terminationssequenz T*_{DIT1},*
   iii) *TbFRDg-R* unter Kontrolle des Promotors P*_{FBA1}* und funktional verbunden mit der Terminationssequenz T*_{ADH1},*
   iv) *AnDCT02* unter Kontrolle des Promotors P*_{COX7}* und funktional verbunden mit der Terminationssequenz T*_{CYC1},* und
c) die folgenden Gene in den LTR-Insertionslokus YGLCτ3 exprimierbar inseriert sind:
   i) *CjFPS1* unter Kontrolle des Promotors P*_{TDH3}* und funktional verbunden mit der Terminationssequenz T*_{RPL15A},*
   ii) *Opgdh* unter Kontrolles des Promotors P*_{TEF1}* und funktional verbunden mit der Terminationssequenz T*_{CYC1},* und
   iii) *ScDAK1* unter Kontrolle des Promotors P*_{ADH2}* und funktional verbunden mit der Terminationssequenz T*_{TPS1}.*

Die erfindungsgemäß gentechnisch veränderte Hefezelle ist so gentechnisch verändert, dass sie eine gegenüber dem Stand der Technik verbesserte Bernsteinsäurebildung aus Glycerol aufweist. Es hat sich überraschend gezeigt, dass die erfindungsgemäße Hefezelle gegenüber der von Xiberras et al. 2020 beschriebenen Hefezelle eine deutlich höhere Ausbeute aufweist. Die erfindungsgemäße Hefezelle zeigt eine maximale Ausbeute von etwa 0,5 g/g Glycerol und damit eine mehr als doppelt so hohe Ausbeute wie die von Xiberras et al. 2020 beschriebene (0,22 g/g Glycerol).

Bei der erfindungsgemäßen Hefezelle ist der Abbau von Glycerol zu Dihydroxyacetonphosphat über den Glycerol-3-Phosphat-Weg (G3P-Weg) blockiert. Dazu ist vorzugsweise das für Glycerolkinase kodierende Gen *GUT1* deletiert. Für eine gegenüber dem Wildtyp verbesserte Glycerol-Aufnahme ist der erfindungsgemäße Stamm darüber hinaus mit dem heterologen Aquaglyceroporin Fps1 aus *Cyberlindnera* (*Pichia*) *jadinii* (*CjFPS1*) ausgestattet. Anders als bei dem von Xiberras et al. 2020 beschriebenen Stamm ist dazu in den LTR-Insertionslokus YGLCτ3 (s. Bai Flagfeldt et al. 2009, Tabelle 2, Nr. 8) eine Expressionskassette inseriert, bei der das Gen für das Aquaglyceroporin (*CjFPS1*) unter der Kontrolle des Promotors P*_{TDH3}* steht und funktional mit der Terminationssequenz T*_{RPL15A}* verbunden ist. In demselben LTR-Insertionslokus, d.h. YGLCτ3, sind bei der erfindungsgemäßen Hefezelle auch Gene für a) eine heterologe Glyceroldehydrogenase aus *Ogataea parapolymorpha* (*Opgdh*) unter Kontrolles des Promotors P*_{TEF1}* und funktional verbunden mit dem Terminator T*_{CYC1}* sowie b) eine homologe Dihydroxyacetonkinase (*ScDAK1*) unter Kontrolle des Promotors P*_{ADH2}* und funktional verbunden mit dem Terminator T*_{TPS1}* inseriert. Die drei Expressionskassetten für *CjFPS1, ScDAK1* und *Opgdh* werden hier zusammen als "DHA-Modul" bezeichnet. In die erfindungsgemäße Hefezelle sind auch Expressionskassetten für ein "SA-Modul", d.h. für Enzyme der reduktiven Umsetzung von Oxalacetat zu Bernsteinsäure, inseriert. Anders als bei dem von Xiberras et al. 2020 beschriebenen Stamm sind Expressionskassetten mit den entsprechenden Genen in den LTR-Insertionslokus YPRCτ3 (s. Bai Flagfeldt et al. 2009, Tabelle 2, Nr. 21) inseriert, wobei Gene für a) eine homologe peroxisomale Malatdehydrogenase (*ScMDH3-R*)*,* die durch Entfernen der peroxisomalen Translokationssequenz im Cytosol verbleibt, unter Kontrolle des Promotors P*_{JEN1}* und funktional verbunden mit dem Terminator T*_{IDP1},* b) eine heterologe Fumarase aus *Rhizopus oryzae* (*RofumR*) unter Kontrolles des Promotors P*_{HOR7}* und funktional verbunden mit dem Terminator T*_{DIT1}* und c) eine heterologe peroxisomale Fumaratreduktase aus *Trypanosoma brucei* (*TbFRDg-R*)*,* die durch Entfernen der peroxisomalen Translokationssequenz im Cytosol lokalisiert bleibt, unter Kontrolle des Promotors P*_{FBA1}* und funktional verbunden mit der Terminator T*_{CYC1}* inseriert sind. Zusätzlich ist in denselben LTR-Insertionslokus, d.h. YPRCτ3, auch eine Expressionskassette für das DCT-02-Gen eines Succinat-Transporters aus *Aspergillus niger* (*AnDCT-02*) unter Kontrolle des Promotors P*_{COX7}* und funktional verbunden mit dem Terminator T*_{CYC1}* inseriert.

Die Integration und entsprechende Expression des Gens für das heterologe Aquaglyceroporin Fps1 aus *Cyberlindnera* (*Pichia*) *jadinii* fördert die Aufnahme von Glycerol, ohne dass hierfür Energie benötigt wird. Die Etablierung oder Stärkung des DHA-Weges durch Einführung der weiteren Komponenten des "DHA-Moduls" und die damit verbundene Expression der hierfür relevanten Enzyme bei gleichzeitiger Blockade des Glycerol-3-phosphat-Wegs führt dazu, dass in die Zelle aufgenommenes Glycerol über den DHA-Weg abgebaut wird. Die Blockade des nativen L-G3P-Weges wurde vorgenommen, um zu verhindern, dass die beteiligte FAD-abhängige Glycerol-3-phosphat-Dehydrogenase, die auf der Außenseite der inneren Mitochondrienmembran lokalisiert ist (Gancedo et al. 1968; Klingenberg 1970), unter aeroben Bedingungen die Elektronen aus der Oxidation von Glycerol-3-phosphat direkt in die mitochondriale Atmungskette mit Sauerstoff als finalem Elektronenakzeptor leitet. Das ist insbesondere dann nachteilig, wenn Elektronen (Reduktionskraft) für eine nachfolgende Herstellung reduzierter Verbindungen benötigt werden. Über den DHA-Weg sollte Glycerol dagegen zumindest theoretisch sowohl unter aeroben als auch unter anaeroben Bedingungen abgebaut werden können. Die Bildung von Succinat wird durch Einführung eines "SA-Moduls" gefördert, indem die reduktive Umsetzung von Oxalacetat zu Succinat, bevorzugt im Cytosol, ermöglicht bzw. verbessert wird. Dies ermöglicht auch die Lenkung des Abbauweges über die CO₂-fixierende Umsetzung von Pyruvat zu Oxalacetet durch die Pyruvat-Carboxylase. Bei der erfindungsgemäßen gentechnisch veränderten Hefezelle ist durch Einführung eines geeigneten heterologen Succinat-Transporters aus *Aspergillus niger* (s. Los et al., 2015) auch der Export von Bernsteinsäure aus der Zelle optimiert. Die erfindungsgemäße Einführung der erforderlichen Expressionskassetten in das Hefegenom an bestimmten Stellen des Genoms in einem bestimmten genregulatorischen Kontext, z.B. mit einer Kombination der erforderlichen Gene und geeigneten Promotoren und Terminatoren, hat zu einer Hefezelle mit einer überraschend hohen Leistung hinsichtlich der Bildung von Bersteinsäure aus Glycerol geführt. Promotoren, die beim Wachstum von *Saccharomyces cerevisiae* auf Glycerol geeignet sind, sind von Ho et al. 2018 beschrieben.

Unter "Glycerol" oder "Glycerin" wird hier die chemische Verbindung Propan-1,2,3-triol (C₃H₈O₃, CAS-Nr. 56-81-5) verstanden.

Unter dem Begriff "Glycerol-3-Phosphat-Weg", "Glycerol-3-Phosphat-Stoffwechselweg", "G3P-Weg" oder auch "Phosphorylierungsweg" wird die Umsetzung von Glycerol über L-Glycerol-3-phosphat (L-G3P, G3P) zu Dihydroxacetonphosphat (DHAP) verstanden.

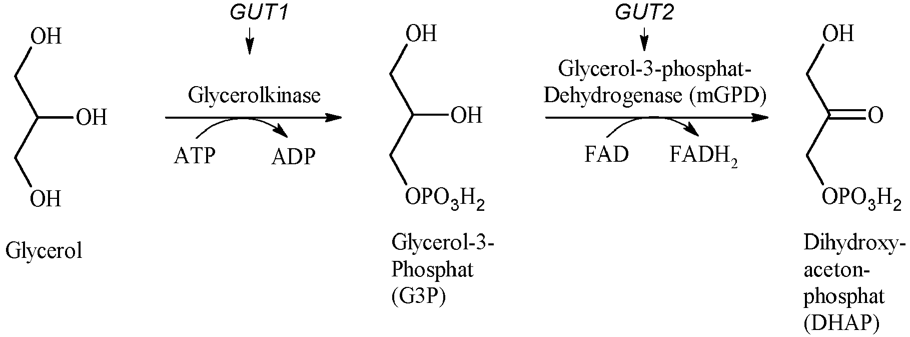

Die Reaktionen werden von der Glycerolkinase und der mitochondrialen (FADabhängigen) Glycerol-3-phosphat-Dehydrogenase (GPD-M, mGPD, EC 1.1.5.3) katalysiert. In der Hefe *Saccharomyces cerevisiae* wird die Glycerolkinase durch das Gen *GUT1,* die mitochondriale Glycerol-3-phosphat-Dehydrogenase durch *GUT2* kodiert.

Unter dem "DHA-Weg", "DHA-Stoffwechselweg" oder "Oxidationsweg" wird die Umsetzung von Glycerol über Dihydroxyaceton (DHA) zu Dihydroxacetonphosphat (DHAP) verstanden.

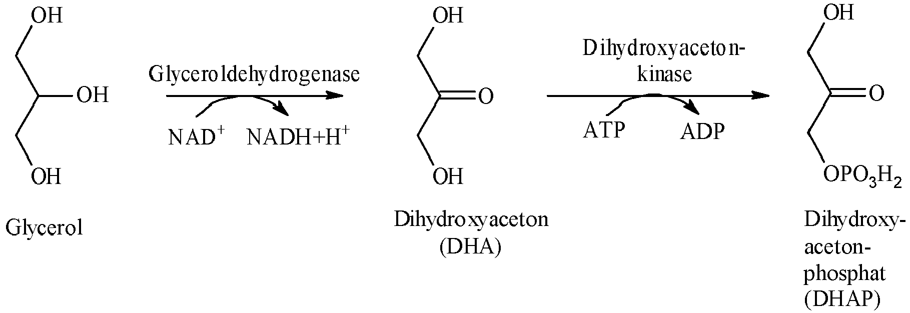

Die Reaktionen werden von der Glyceroldehydrogenase (EC 1.1.1.6) und der Dihydroxyacetonkinase (EC 2.7.1.29) katalysiert.

Mit dem Begriff "DHA-Modul" wird hier eine Zusammenstellung von Expressionskonstrukten für ein Aquaglyceroporin (Fps1) und für Enzyme verstanden, die für den DHA-Weg erforderlich oder vorteilhaft sind, d.h. für Glyceroldehydrogenase und Dihydroxyacetonkinase.

Mit dem Ausdruck "reduktive Umsetzung von Oxalacetat zu Succinat" wird der Weg der Umsetzung von Oxalacetat zu Succinat verstanden:

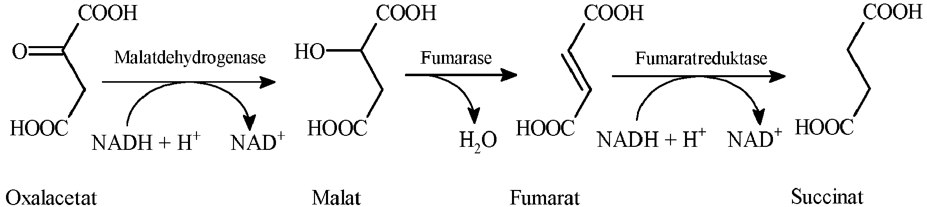

Der Begriff "SA-Modul" bezeichnet hier eine Zusammenstellung von Expressionskonstrukten für Enzyme, die für die reduktive Umsetzung von Oxalacetat zu Succinat erforderlich sind, d.h. Malatdehydrogenase, Fumarase (Fumarat-Hydratase) und Fumaratreduktase.

Der Begriff "heterolog" wird hier in seiner dem Fachmann bekannten Bedeutung verwendet, und bezieht sich auf die fremde Herkunft eines Elements, beispielsweise eines Enzyms oder anderen Proteins, bzw. eines dafür kodierenden Gens. "Fremd" bedeutet, dass das Element so in der Zielzelle nicht vorkommt, und beispielsweise aus einer Zelle oder einem Organismus mit abweichender genetischer Ausstattung, beispielsweise einem Organismus einer anderen Art, stammt.

Der Begriff "homolog" wird hier in Bezug auf ein Enzym, Protein oder Gen gegebenenfalls verwendet, um dieses als ein natives, d.h. in der Zielzelle so auch natürlicherweise vorkommendes Enzym Protein oder Gen zu kennzeichnen, im Gegensatz zu einem heterologen Enzym/Protein/Gen.

Der Begriff "LTR-Insertionslokus" bezeichnet eine Insertionsstelle innerhalb eines "Long Terminal Repeat". LTRs sind DNA-Wiederholungseinheiten ("repeats") im Genom von Eukaryoten, z.B. im Hefegenom, die LTR-Elemente (z.B. Gene oder Pseudogene) flankieren, und für die Transposition der LTR-Elemente innerhalb des Genoms erforderlich sind. Unter dem Begriff "LTR-Insertionslokus YPRCτ3" wird eine Insertionsstelle innerhalb des LTRs YPRCτ3 auf Chromosom XVI im Genom von *Saccharomyces cerevisiae* verstanden (s. Bai Flagfeldt et al. 2009, Tabelle 2, Nr. 21). Unter dem "LTR-Insertionslokus YGLCτ3" wird eine Insertionsstelle innerhalb des LTRs YGLCτ3 auf Chromosom VII im Genom von *Saccharomyces cerevisiae* verstanden (s. Bai Flagfeldt et al. 2009, Tabelle 2, Nr. 8).

Unter einem "Promotor" wird eine regulatorische DNA-Sequenz in der Nähe des Transkriptionsstarts einer kodierenden Sequenz, z.B. eines Gens, verstanden, an die Proteine (inklusive RNA Polymerase) binden, die die Transkription der stromabwärts vom Promotor liegende DNA in eine RNA initiieren. Der Begriff umfasst auch DNA-Sequenzen in der Nähe des Transkriptionsstarts einer zu transkribierenden DNA-Sequenz, die aufgrund ihrer Interaktion mit DNA und/oder Proteinkomponenten eine regulierende, beispielsweise aktivierende Funktion auf die Transkription haben. Der Begriff umfasst auch regulatorische DNA-Sequenzen, die den Transkriptionsstart umfassen. Der Ausdruck "unter der Kontrolle des Promotors X" in Bezug auf ein Gen, beispielsweise ein Gen, das ein Enzym kodiert, bedeutet, dass dem Gen der Promotor X vorangestellt ist und das Gen unter dessen Kontrolle exprimiert wird.

Unter einer "Terminationssequenz" oder einem "Terminator" wird eine regulatorische DNA-Sequenz verstanden, die die Termination der RNA-Transkription signalisiert. Die Ausdrücke "funktional verbunden mit der Termationssequenz X" oder "funktional verbunden mit dem Termator X" in Bezug auf ein Gen, beispielsweise ein Gen, das ein Enzym kodiert, bedeutet, dass die Terminationssequenz (der Terminator) X die Termination der RNA-Transkription des Gens signalisiert.

Unter einem "Aquaglyceroporin", hier gegebenenfalls auch synonym als "Glycerolaufnahme-Channelprotein" oder kurz "Glycerol-Channel" bezeichnet, wird ein Transmembranprotein verstanden, das den Transport von Glycerol in eine Zelle, beispielsweise eine *Saccharomyces*-Zelle*,* mittels erleichterter Diffusion ermöglicht.

Unter "Glyceroldehydrogenase" (kurz GDH) wird hier eine cytosolische Glycerol:NAD⁺-Oxidoreduktase (EC 1.1.1.6) verstanden, welche die Oxidation von Glycerol zu Dihydroxyaceton mittels NAD⁺ als Kofaktor katalysiert. Unter einer "heterologen Glyceroldehydrogenase" wird eine cytosolische Fremd-Glyceroldehydrogenase verstanden, d.h. eine aus einem anderen Organismus, z.B. einer anderen Art, Gattung etc., stammende Glyceroldehydrogenase, die jedoch dieselbe Reaktion katalysiert, gegebenenfalls unter Nutzung eines anderen Kofaktors (insbesondere NADP⁺). Die Abkürzung *"Opgdh"* bezeichnet ein Gen, das für die Glyceroldehydrogenase aus *Ogataea parapolymorpha* kodiert. Der Begriff "cytosolisch" in Bezug auf ein Enzym bezieht sich auch auf Enzyme, die in ihrer Struktur so verändert ("retargeted") sind, beispielsweise durch Entfernen einer Translokationssequenz, die ansonsten dafür sorgen würde, dass das Enzym in ein Zellorganell, z.B. das Peroxisom, transloziert wird, im Cytosol verbleibt.

Unter dem Begriff "Dihydroxyacetonkinase" oder "DHA-Kinase" (kurz DAK) wird hier eine ATP:Dihydroxyaceton-Phosphotransferase (EC 2.7.1.29) verstanden, die die Phosphorylierung von Dihydroxaceton mittels ATP katalysiert. Die Abkürzung *"ScDAK1"* bezeichnet das Gen für Dihydroxyacetonkinase 1 aus *Saccharomyces cerevisiae.*

Unter "Pyruvat-Carboxylase" (EC 6.4.1.1, PYC) wird hier ein Enzym verstanden, das die Bildung von Oxalacetat durch Addition von Kohlenstoffdioxid an Pyruvat katalysiert. Die katalytische Reaktion verbraucht ATP. *Saccharomyces cerevisiae* besitzt zwei Isoenzyme von PYC, wobei "Pyc2" die Pyruvat-Carboxylase 2 bezeichnet und *PYC2* das dafür kodierende Gen. Die Abkürzung "*PYC2*ₒₑ" bezieht sich auf ein überexprimiertes Pyc2-Gen.

Unter einer "cytosolischen Malatdehydrogenase" (EC 1.1.1.37, MDH) wird ein im Cytoplasma vorkommendes Enzym verstanden, das die Reduktion von Oxalacetat zu Malat mittels NADH katalysiert. *Saccharomyces cerevisiae* besitzt mehrere Isoenzyme von MDH (Mdh1, Mdh2, Mdh3). Der Begriff schließt auch peroxisomale Malatdehydrogenasen ein, die so verändert sind, z.B. durch Entfernung entsprechender Signalsequenzen, dass sie im Cytosol verbleiben und nicht in Peroxisomen transloziert werden. Die Abkürzung "*ScMDH3-R*" bezeichnet das Gen für ein zum Cytosol "retargeted" (das R steht für "retargeted) Malatdehydrogenase-Isoenzym Mdh3 aus *Saccharomyces cerevisiae,* das in seiner Target-Sequenz so verändert wurde, dass es nicht mehr peroxisomal, sondern cytosolisch lokalisiert ist.

Unter einer "Fumarase" (EC 4.2.1.2, Fumarat-Hydratase, (S)-Malat-Hydrolyase) wird ein Enzym des Tricarbonsäurezyklus verstanden, das die Bildung von Fumarat aus Malat katalysiert. Die Abkürzung "*RofumR*" bezeichnet das Gen für eine Fumarase aus *Rhizopus oryzae.*

Unter einer "Fumaratreduktase" (EC 1.3.1.6, EC 1.3.5.4, EC:1.3.5.1, FRD) wird ein Enzym des Tricarbonsäurezyklus verstanden, das Fumarat zu Succinat reduziert, vorzugsweise eine NADH-abhängige Fumaratreduktase (EC 1.3.1.6). Die Abkürzung "*TbFRDg-R*" bezeichnet ein Gen für eine zum Cytosol "retargeted" Fumaratreduktase aus *Trypanosoma brucei.*

Unter einem "Gen" wird hier, soweit sich aus dem Zusammenhang nicht etwas anderes ergibt, ein DNA-Abschnitt verstanden, der die kodierende Sequenz für ein Protein, z.B. ein Enzym, bildet oder umfasst, ausgenommen regulatorische Sequenzen wie Promotoren und Terminatoren.

Mit der Abkürzung "Ubr2" wird die cytoplasmatische Ubiquitin-Protein-Ligase (E3) (YLR024C, SGD ID: SGD:S000004014) bezeichnet, die Abkürzung "*UBR2*" bezieht sich auf das dafür kodierende Gen. Die Formulierung, wonach das UBR2-Allel eines Hefestammes gegen das Allel *UBR2_{CBS 6412-13A}* ausgetauscht ist, bedeutet, dass im Genom der haploiden Hefezelle an der Stelle des endogenen UBR2-Allels lediglich das Allel *UBR2_{CBS 6412-13A}* vorhanden ist, d.h. das Allel des auf Gycerol wachsenden haploiden *Saccharomyces*-Stammes CBS 6412-13A (s. Swinnen et al. 2013, Swinnen et al. 2016, Klein et al., 2016, Xiberras et al. 2020, Ho et al. 2017), einschließlich des zugehörigen Promotors und Terminators. Die Formulierung, wonach das *UBR2*-Allel eines Hefestammes gegen das Allel *UBR2_{CEN.PK113-7D JL1}* ausgetauscht ist, bedeutet, dass im Genom der Hefezelle an der Stelle des endogenen *UBR2*-Allels lediglich das Allel *UBR2_{CEN.PK113-7D JL1}* vorhanden ist, d.h. das Allel des auf Gycerol wachsenden evolvierten haploiden *Saccharomyces*-Stamms CEN.PK113-7D JL1 bzw. der evolvierten Stämme CEN.PK113-1A PW-1 oder CEN.PK113-1A PW-2 (s. Ochoa-Estopier 2011, Ho et al. 2017).

Unter "C₄-Dicarbonsäuren" werden Dicarbonsäuren, d.h. Carbonsäuren mit zwei CarboxyGruppen (-COOH), mit 4 C-Atomen verstanden. Beispielse für C₄-Dicarbonsäuren sind Bernsteinsäure (Butandisäure, Succinylsäure, C₄H₆O₄), Fumarsäure (trans-Butendisäure, C₄H₄O₄) und Äpfelsäure (2-Hydroxybutandisäure, C₄H₅O₅). Gegebenenfalls werden hier synonym auch die Begriffe, die überlicherweise die Salze der Dicarbonsäuren bezeichnen, verwendet. Für Bernsteinsäure wird beispielsweise synonym auch "Succinat", für Fumarsäure "Fumarat" und für Äpfelsäure "Malat" verwendet. Für Bernsteinsäure wird hier gegebenenfalls auch die Abkürzung "SA" (für engl. "succinic acid", Bernsteinsäure), verwendet.

Unter einem "C₄-Dicarbonsäure-Transporterʺ wird ein Transmembranprotein verstanden, das den Transport einer C₄-Dicarbonsäure wie beispielsweise Bernsteinsäure, Äpfelsäure, oder Fumarsäure über eine Zellmembran hinweg, d.h. aus einer Zelle, beispielsweise einer *Saccharomyces*-Zelle*,* heraus oder in die Zelle hinein ermöglicht. Der Begriff schließt auch Proteine ein, die neben C₄-Dicarbonsäuren weitere Moleküle transportieren. Unter einem "Succinat"-Transporter wird ein "C₄-Dicarbonsäure-Transporter" verstanden, der den Transport von Bernsteinsäure oder dessen Salz aus der Zelle heraus erleichtert oder ermöglicht. Die Abkürzungen "DCT-02" oder "AnDCT-02" bezeichnen einen C₄-Dicarbonsäure-Transporter aus *Aspergillus niger,* der u.a. Succinat transportiert, "*AnDCT-*02" das dafür kodierende Gen.

Unter "Expression" wird hier die Umsetzung einer genetischen Information in ein Produkt verstanden, beispielsweise die Bildung eines Proteins oder einer Nukleinsäure anhand der genetischen Information. Der Begriff umfasst insbesondere die Biosynthese von Proteinen anhand der genetischen Information einschließlich vorangehender Prozesse wie der Transkription, d.h. der Bildung von mRNA auf Basis einer DNA-Vorlage. Sofern der Ausdruck hier in Bezug auf ein Protein verwendet wird, beispielsweise in der Form "Protein x wird exprimiert" oder "Protein x wird überexprimiert", ist damit gemeint, dass das zugehörige Proteingen exprimiert bzw. überexprimiert wird.

Unter "Überexpression" wird eine Expression eines Gens verstanden, die im Vergleich zu einer Expression desselben Gens stärker ist, d.h. zu mehr Genprodukt führt. In der Regel wird hier von Überexpression gesprochen, wenn ein natives, d.h. ein in der Zelle natürlicherweise vorkommendes, Genprodukt stärker als unter natürlichen Bedingungen exprimiert wird. Eine Überexpression kann beispielsweise durch Einführung zusätzlicher Kopien des für das Genprodukt kodierenden Gens in eine Zelle und/oder die Ersetzung des natürlichen Promotors, unter dessen Kontrolle die Expression des Gens erfolgt, durch einen stärkeren Promotor bewirkt werden. Der Begriff "zusätzliche Kopien" kann auch beinhalten, dass mindestens ein für das Genprodukt kodierendes heterologes Gen eingeführt wird.

Der Ausdruck, dass ein heterologes Protein/Enzym exprimiert wird, schließt ein, dass das Protein/Enzym im Vergleich zum durchschnittlichen Expressionsniveau des Proteins/Enzyms in der Herkunftszelle in der Zielzelle unter ansonsten gleichen Bedingungen überexprimiert wird. Der Ausdruck schließt auch ein, dass das Protein/Enzym unter Kontrolle eines beliebigen Promotors exprimiert wird, also auch eines Promotors, der von dem Promotor verschieden sein kann, der in der Herkunftszelle mit dem Protein/Enzym verknüpft ist oder der in der Zielzelle mit einem etwaigen orthologen Protein/Enzym verknüft ist.

Unter dem Begriff der "Blockade" oder des "Blockierens" eines Stoffwechselweges, d.h. einer Kette von Reaktionen mit einer Ausgangsverbindung, mindestens einem Zwischenprodukt und einem Endprodukt (das selbst auch wieder die Ausgangsverbindung für einen anderen Stoffwechselweg sein kann) wird hier verstanden, dass die Bildung zumindest des ersten Zwischenproduktes des Weges unterbunden oder wesentlich vermindert ist. "Blockade" oder "Blockieren" bedeutet hier insbesondere, dass im Vergleich zu einer Hefezelle, bei der der Stoffwechselweg nicht blockiert ist, die Bildung des ersten Zwischenprodukts um mindestens 75 %, vorzugsweise mindestens 80 %, mindestens 85 %, mindestens 90 %, mindestens 95 % oder mindestens 98 % vermindert ist. In Bezug auf den Glycerol-3-Phosphat-Stoffwechselweg wird unter einer Blockade daher verstanden, dass zumindest der Abbau von Glycerol zu Glycerol-3-Phosphat durch die Glycerolkinase (kodiert durch *GUT1*) blockiert ist. Der Begriff schließt auch ein, dass sämtliche Zwischenschritte auf dem Weg zum Endprodukt blockiert sind, beispielweise durch Deletieren oder Mutieren der Gene sämtlicher an dem Weg beteiligter Enzyme, so dass kein funktionsfähiges Genprodukt oder nur ein in seiner Funktion wesentlich eingeschränktes Genprodukt, d.h. Enzym, resultiert. Der Begriff kann auch bedeuten, dass die Bildung von funktionsfähigem Enzym um die oben genannten Prozentzahlen vermindert ist, indem beispielsweise die natürlichen Promotoren durch entsprechend schwächere ausgetauscht werden, oder dass nur eine solche Menge eines in seiner Funktionalität eingeschränkten Enzyms gebildet wird, dass die Bildung des Zwischenproduktes wie oben definiert vermindert ist. Mittel zur Modifizierung eines Stoffwechselweges im Wege des "Metabolic Engineering" sind dem Fachmann gut bekannt (s. z.B. Nevoigt 2008; Mapelli 2014). Beispielsweise kann ein Stoffwechselweg durch Deletieren oder Deaktivieren eines Gens oder eines für die Regulation eines Gens relevanten DNA-Abschnitts, beispielsweise mittels zielgerichteter Mutation, blockiert werden. Eine Modifikation dahingehend, dass ein heterologes Enzym exprimiert wird, kann durch dem Fachmann bekannte gentechnische Verfahren erreicht werden, beispielsweise indem ein geeigneter Expressionsvektor mit einem das Enzym kodierenden Gen (zusammen mit einem geeigneten Promoter und Terminator) in die Zelle eingebracht wird.

Wenn hier der Ausdruck "Deaktivieren" in Zusammenhang mit einem Enzym verwendet wird, bedeutet dies, dass im Vergleich zu einer Wildtypzelle zumindest eine deutlich verminderte Aktivität des entsprechenden Enzyms vorliegt. Insbesondere bedeutet der Ausdruck, dass die Enzymaktivität im Vergleich zu einer Wildtypzelle um mindestens 75 %, vorzugsweise mindestens 80 %, mindestens 85 %, mindestens 90 %, mindestens 95 %, 96 %, 97 %, mindestens 98 % oder mindestens 99 % vermindert ist. Bevorzugt ist im Wesentlichen keine Enzymaktivität vorhanden. Eine Deaktivierung eines Enzyms kann beispielsweise durch Deletieren oder Mutieren des das Enzym kodierenden Gens erfolgen. Methoden zur Deaktivierung von Enzymen sind dem Fachmann bekannt. In Zusammenhang mit der vorliegenden Erfindung ist der Ausdruck insbesondere so zu verstehen, dass eine von einem Enzym durchgeführte Reaktion im Vergleich zu einer Wildtypzelle in deutlich vermindertem Umfang stattfindet, vorzusweise im Vergleich zu einer Wildtypzelle in einem um mindestens 75 %, vorzugsweise mindestens 80 %, mindestens 85 %, mindestens 90 %, mindestens 95 %, 96 %, 97 %, mindestens 98 % oder mindestens 99 % verminderten Umfang. Der Begriff "Enzym" umfasst hier auch sämtliche Isoformen des Enzyms.

Der Begriff "Fermentation" oder "fermentativ" bedeutet, dass ATP ausschließlich oder zumindest weit überwiegend über Substratkettenphosphorylierung und nicht durch oxidative Phosphorylierung, d.h. durch Elektronentransportketten, gewonnen wird. Im Falle eines fermentativen Stoffwechsels werden die aus dem Elektronendonor (zum Beispiel Glycerol) stammenden Elektronen nicht auf Sauerstoff, sondern auf Zwischenprodukte des Metabolismus übertragen was zur Bildung von Fermentationsprodukten führt.

Unter einer "biotechnologischen Herstellung" eines Produktes, z.B. Glycerol, wird die Herstellung eines Produktes durch biologische Mittel, beispielsweise durch lebende Zellen, zum Beispiel Hefezellen, durch Enzyme oder lebende Organismen, verstanden.

Der Ausdruck "einzige Kohlenstoffquelle", beispielsweise in Bezug auf die Nutzung von Glycerol durch die erfindungsgemäße Hefezelle, bedeutet im Kontext der Erfindung, dass Glycerol die einzige organische Kohlenstoffquelle ist und schließt nicht aus, dass durch die Zelle zusätzlich auch Kohlendioxid aus der Luft oder aus anderen Stoffwechselprozessen fixiert, d.h. in eine organische Verbindung eingebaut wird.

Mit dem Begriff "CEN.PK-Familie" wird eine isogene Familie von *Saccharomyces-cerevisiae*-Stämmen verstanden (s. z.B. Entian und Kötter 2007; van Dijken et al. 2000; Nijkamp, J.F. et al. 2012; EUROPEAN SACCHAROMYCES CEREVISIAE ARCHIVE FOR FUNCTIONAL ANALYSIS (EUROSCARF), Zugriffsnummter 30000D). Das bekannteste Beispiel eines Stammes aus dieser Familie ist der Stamm CEN.PK113-7D. Informationen zum Genom dieses Stammes sind unter der GenBank-Zugriffsnummer JRIV00000000 verfügbar (s. z.B. https://www.yeastgenome.org/strain/CEN.PK). Ein anderes Beispiel ist der Stamm CEN.PK113-1A, der genau wie CEN.PK113-7D ebenfalls prototroph ist und sich von letzterem nur durch den Kreuzungstyp unterscheidet (CEN.PK113-1A ist Mat alpha und CEN.PK113-7D ist Mat a). Die Formulierung, wonach ein bestimmter Stamm bzw. eine Variante davon einen "genomischen CEN.PK-Hintergrund" oder kürzer einen "CEN.PK-Hintergrund" hat, bedeutet, dass dieser Stamm zu der CEN.PK-Familie gehört und/oder von einem Mitglied dieser Familie abgeleitet ist und entsprechend die allgemeine genomische Ausstattung dieser Familie aufweist. Wenn hier in Bezug auf eine Hefezelle, beispielsweise eine erfindungsgemäß veränderte Zelle, auf den genomischen Hintergrund eines Stammes aus der CEN.PK-Familie, z.B. CEN.PK113-1A, hingewiesen wird, bedeutet dies, dass die veränderte Zelle, abgesehen von den spezifischen genomischen Änderungen, die an der Zelle vorgenommen wurden, im Wesentlichen isogen zu diesem Stamm ist.

Der Begriff "Bicarbonat" bezieht sich auf Hydrogencarbonat (HCO₃⁻).

Die erfindungsgemäße Hefezelle weist vorzugsweise einen genomischen CEN.PK-Hintergrund, besonders bevorzugt einen genomischen CEN.PK113-1A-Hintergrund auf.

In einer besonders bevorzugten Ausführungsform der erfindungsgemäßen Hefezelle ist die Hefezelle ferner dahingehend gentechnisch verändert, dass
d) das *UBR2* Allel gegen das Allel *UBR2_{CBS 6412-13A},* das Allel *UBR2_{CEN.PK113-7DJL1},* das Allel *UBR2_{CEN.PK113}-_{1A PW-1}* oder das Allel *UBR2_{CEN.PK113-1A PW-2}* ausgetauscht ist.

Der Austausch des UBR2-Allels gegen das Allel *UBR2_{CBS 6412-13A}* (kurz auch "*UBR2*_{CBS}" oder "*UBR*_{CBS}") verbessert die Glycerolnutzung durch *Saccharomyces,* zumindest von Hefezellen mit einem genomischen CEN.PK-Hintergrund (s. Swinnen et al. 2013, Swinnen et al. 2016, Xiberras et al. 2020). Das Allel *UBR2_{CBS 6412-13A}* zeichnet sich dadurch aus, dass es ein Volllängen-Ubr2 kodiert, d.h. ein Ubr2-Protein mit 1872 Aminosäuren, und nicht das bei Mitgliedern der CEN.PK-Familie häufige trunkierte Ubr2-Protein (s. Ho et al. 2017). Gleiches gilt auch für das Allel *UBR2_{CEN.PK113-7D JL1}* und die Allele *UBR2_{CEN.PK113-1A PW-1}* und *UBR2_{CEN.PK113-1A PW-2}* (s. Ochoa-Estopier 2011, Ho et al. 2017).

In einer bevorzugten Ausführungsform handelt es sich bei der erfindungsgemäßen Hefezelle um eine Hefezelle, die am 10.01.2021 nach dem Budapester Vertrag über die internationale Anerkennung der Hinterlegung von Mikroorganismen für die Zwecke von Patentverfahren unter der Zugriffsnummer 58096 bei der belgisch koordinierten Sammlung von Mikroorganismen (Belgian Coordinated Collections of Microorganisms, BCCM) hinterlegt worden ist.

In einer weiter bevorzugten Ausführungsform der erfindungsgemäßen Hefezelle ist diese zusätzlich dahingehend gentechnisch verändert, dass die endogene (homologe) Pyruvatcarboxylase 2, Pyc2, bzw. das dafür codierende Gen *(PYC2),* überexprimiert wird. Es hat sich gezeigt, dass eine derart zusätzlich veränderte Ausführungsform einer erfindungsgemäßen Hefezelle die gleiche Ausbeute zeigt wie eine erfindungsgemäße Hefezelle, bei der Pyc2 nicht überexprimiert ist, diese Menge Bernsteinsäure jedoch in kürzerer Zeit bildet, d.h. eine höhere Glycerolverbrauchsrate aufweist. Eine Überexpressionskassette umfassend *PYC2,* bevorzugt unter der Kontrolle des *HOR7-*Promotors und des *TPS1*-Terminators, ist vorzugsweise an Position XI-3 (Chromosom XI: 93378..94567, Jessop-Fabre et al. 2016) inseriert.

Die erfindungsgemäße Hefezelle ist bevorzugt haploid.

In einem weiteren Aspekt betrifft die vorliegende Erfindung auch ein Verfahren zur biotechnologischen Herstellung von Bernsteinsäure aus Glycerol, wobei eine erfindungsgemäß gentechnisch veränderte Hefezelle nach dem oben beschriebenen Aspekt in einem glycerolhaltigen Kulturmedium unter geeigneten Bedingungen kultiviert und Bernsteinsäure aus dem Kulturmedium gewonnen, d.h. von dem Kulturmedium abgetrennt wird. Vorzugsweise erfolgt die biotechnologische Herstellung fermentativ. Besonders bevorzug ist Glycerol bei dem erfindungsgemäßen Verfahren einzige Energiequelle und einziger Elektronendonor, und Glycerol ist die einzige Kohlenstoffquelle. "Geeignete Bedingungen" sind dem Fachmann bekannt und beziehen sich beispielsweise auf Umgebungsfaktoren wie Temperatur, pH-Wert, Medienzusammensetzung etc, unter denen die erfindungsgemäße Hefezelle möglichst dauerhaft lebensfähig bleibt und Bernsteinsäure bildet.

In einer bevorzugten Ausführungsform des erfindungsgemäßen Verfahrens erfolgt Kultivierung der erfindungsgemäßen Hefezelle in dem glycerolhaltigen Kulturmedium in Gegenwart von dem Kulturmedium zugegebenem CaCO₃ oder unter Zufuhr von gasförmigem Kohlendioxid in das Kulturmedium. Dies ist besonders vorteilhaft bei der Kultivierung einer erfindungsgemäßen Hefezelle, die dahingehend gentechnisch verändert ist, dass die Pyruvatcarboxylase 2, *Pyc2,* überexprimiert wird. Titer und Ausbeute an produzierten Dicarbonsäuren können auf diese Weise weiter verbessert werden. Es wird angenommen, dass eine erhöhte Bicarbonat-Konzentration im Medium die Aufnahme von CO₂ durch die Zelle verstärkt, das bei der durch die Pyruvatcarboxylase katalysierten Reaktion fixiert werden kann. Entsprechend ist dies besonders vorteilhaft bei einer Hefezelle, bei der die Pyruvatcarboxylase überexprimiert wird.

Die Erfindung wird im Folgenden anhand von Ausführungsbeispielen und der beigefügten Zeichnungen beispielhaft näher erläutert.

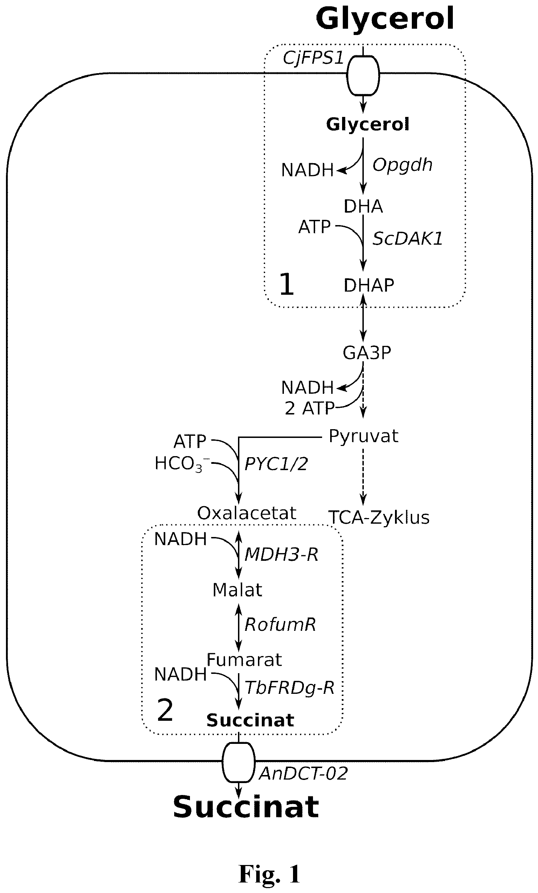

Fig. 1 Vereinfachte schematische Darstellung von an einer Ausführungsform einer erfindungsgemäßen gentechnisch veränderten Hefezelle vorgenommenen Modifikationen für einen fermentativen Abbau von Glycerol zu Succinat. 1 = "DHA-Modul", 2 = "SA-Modul", DHA = Dihydroxyaceton, DHAP Dihydroxyacetonphosphat, GA3P = Glycerinaldehyd-3-Phosphat.

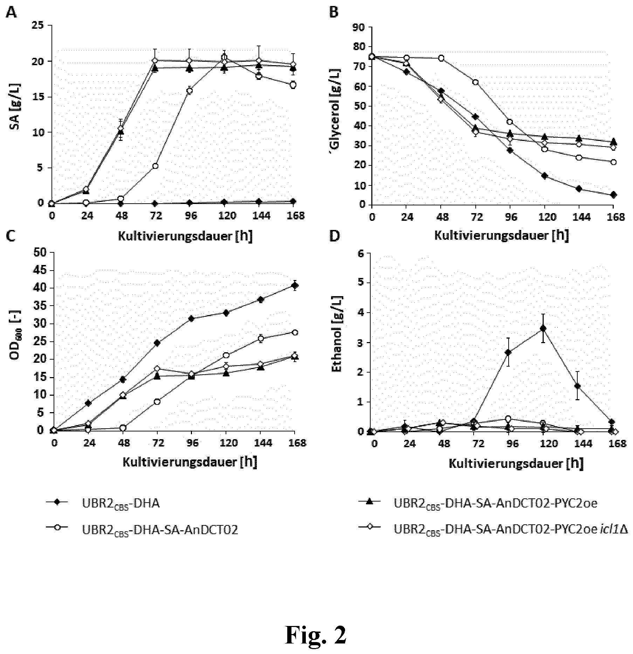

Fig. 2 Produktion von Bernsteinsäure ("SA"), Fig. 1A und Biomasse (Fig. 1C) sowie Verbrauch von Glycerol (Fig. 1B) durch die rekonstruierten (erfindungsgemäßen) Stämme *UBR2*_{CBS}-DHA-SA-*An*DCT-02 und *UBR2*_{CBS}-DHA-SA-*An*DCT-02-*PYC2*ₒₑ sowie die Variante *UBR2*_{CBS}-DHA-SA-*An*DCT-02-*PYC2*ₒₑ *icl1*Δ*.* Die Kultivierung wurde in 500-ml-Schüttelkolben-Batch Kultivierung durchgeführt, die mit 100 ml synthetischem Glycerolmedium bei pH 4 unter Verwendung von Harnstoff als Stickstoffquelle gefüllt waren. Die HPLC-Analyse wurde verwendet, um die Konzentrationen von Glycerol und SA im Kulturüberstand zu bestimmen. Die Biomasseakkumulation wurde durch Messung der optischen Dichte bei 600 nm (OD₆₀₀) bestimmt. Mittelwerte und Standardabweichungen wurden aus drei biologischen Replikaten bestimmt.

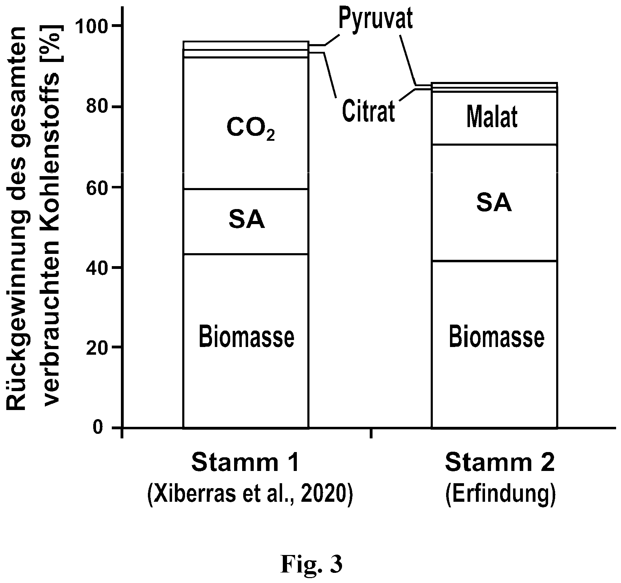

Figur 3 Verbleib des verbrauchten Kohlenstoffs bei Anzucht eines Hefestammes aus dem Stand der Technik (Stamm 1: *UBR2*_{CBS}-DHA-SA-*An*DCT-02 gemäß Xiberras et al., 2020) und eines erfindungsgemäß gentechnisch veränderten Hefestammes (Stamm 2: *UBR2*_{CBS}-DHA-SA-*An*DCT-02-PYC2oe gemäß der vorliegenden Erfindung). Die Daten auf der linken Seite stammen von Xiberras et al. (2020), die Daten auf der rechten Seite repräsentieren das Schicksal des verbrauchten Kohlenstoffs in der produktiven Phase (0-71,75 h) der Batch-Bioreaktor-Kultivierung des erfindungsgemäßen Hefestamms (Stamm 2). Der Gesamtkohlenstoffverbrauch wurde durch Addition des Nettoverbrauchs an CO₂ und Glycerin ermittelt. Die Bildung von Biomasse und Metaboliten wurde durch Zelltrockengewicht und HPLC-Analyse bestimmt. Verdünnungseffekte aufgrund der KOH-Zugabe zur pH-Kontrolle und Entnahme des Probenvolumens wurden berücksichtigt. Die Daten repräsentieren den Mittelwert zweier Experimente und stellen den Kohlenstoff dar, der in verschiedenen Produkten endet, als Anteil des gesamten verbrauchten Kohlenstoffs. Aufgrund technischer Schwierigkeiten wurden die CO₂-Protokollierungsdaten der ersten Stunde eines Versuchsansatzes weggelassen.

Figur 1 stellt vereinfacht gentechnische Veränderungen an einer Ausführungsform einer erfindungsgemäßen gentechnisch veränderten Hefezelle dar. Die Hefezelle ist so verändert, dass Glycerol über ein heterologes Aquaglyceroporin (CjFps1) in die Zelle aufgenommen und Bernsteinsäure über einen heterologen Succinat-Transporter (*An*DCT-02) wieder ausgeschieden wird. Auf dem Weg dorthin wird Glycerol über den DHA-Weg mittels eines in die Zelle in spezifischer Weise eingebrachten "DHA-Moduls" 1 und eines ebenfalls in die Zelle in spezifischer Weise eingebrachten "SA-Moduls" zu Succinat umgesetzt.

Trotz Ähnlichkeiten mit der von Xiberras et al. 2020 beschriebenen Hefezelle unterscheidet sich die erfindungsgemäße Hefezelle deutlich von dieser. In der folgenden Tabelle 1 sind Unterschiede zwischen dem von Xiberras et al. 2020 beschriebenen Hefestamm und der erfindungsgemäßen Hefezelle zusammengefasst.

**Tabelle 1. Vergleich von gentechnischen Veränderungen am Genom des in Xiberras et al. 2020 beschriebenen Hefestamm und denen der erfindungsgemäßen Hefezelle. Änderungen bei der Erfindung gegenüber Xiberras et al. sind unterstrichen.**

| | | | |
|---|---|---|---|
| Xiberras et al. 2020 | | Erfindung | |
| DHA-Modul | | DHA-Modul | |

| Kassette | Position | Kassette | Position |
|---|---|---|---|
| P*_{TEF1}*-*Opgdh*-T_{C*YC1*} | *gut1* | P*_{TEF1}-Opgdh*-T*_{CYC1}* | *YGLCτ3* |
| P*_{ACT1}-ScDAK1-T_{TPS1}* | *gut1* | P*_{ADH2}-ScDAK1*-T*_{TPS1}* | *YGLCτ3* |
| P*_{PGK1}-CjFPS1-T_{RPL15A}* | *gut1* | *P_{TDH3}-CjFPS1*-T*_{RPL15A}* | *YGLCτ3* |
| P*_{TDH3}*-*ScDAK1*-T*_{IDP1}* | *gut1* | loxP-P*_{TEF1}*-ble-T*_{TEF1}*-loxP | *gut1* |
| SA module + An-DCT-02 transporter | | SA-Modul + An-DCT-02-Transporter | |

| Kassette | Position | Kassette | Position |
|---|---|---|---|
| P*_{PGK1}-ScMDH3-R*-T*_{IDP1}* | *YGLCτ3* | *P_{JEN1}-ScMDH3-R*-T*_{IDP1}* | *YPRCτ3* |
| P*_{TEF1}-RofumR*-T*_{RPL15A}* | *YGLCτ3* | P*_{HOR7}-RofumR*-T*_{DIT1}* | *YPRCτ3* |
| P*_{TDH3}-TbFRDg-R*-T*_{CYC1}* | *YGLCτ3* | P*_{FBA1}-TbFRDg-R*-T*_{ADH1}* | *YPRCτ3* |
| P*_{ENO2}-AnDCT2-02*-T*_{DIT1}* | *YPRCτ3* | P*_{COX7-}AnDCT-02*-T*_{CYC1}* | *YPRCτ3* |

Der Stamm gemäß Xiberras et al. 2020 wird hier auch mit "*UBR2*_{CBS}-DHA-SA-*An*DCT-02" bezeichnet. Sofern sich hier nicht eindeutig aus dem Zusammenhang etwas anderes ergibt, wird hier unter einem "rekonstruierten" Stamm, z.B. einem rekonstruierten Stamm ʺ*UBR2*_{CBS}-DHA" oder einem "rekonstruierten *UBR2*_{CBS}-DHA-SA-*An*DCT-02" ein erfindungsgemäßer Stamm mit entsprechenden in Tabelle 1 in der rechten Spalte angegebenen gentechnischen Veränderungen gegenüber dem von Xiberras et al. beschriebenen Stamm (Tabelle 1, linke Spalte) verstanden. "Rekonstruiert" bedeutet hier somit nicht einen identischen Nachbau, sondern eine "analoge" Neukonstruktion, d.h. eine Variante mit Genomänderungen im Vergleich zum Stand der Technik (Xiberras et al. 2020), die lediglich gleiche funktionelle, beispielsweise regulatorische Elemente betreffen. Ein ebenfalls erfindungsgemäßer, jedoch durch Überexpression von Pyc2 weiter gentechnisch veränderter Stamm wird als "*UBR2*_{CBS}-DHA-SA-*An*DCT-02 *PYC2*ₒₑ" bezeichnet.

### Beispiel

Im Folgenden werden die Herstellung einer Ausführungsform eines erfindungsgemäßen Hefestamms sowie Ergebnisse zum Glycerolabbau dargestellt.

### Stämme, Plasmide und Kultivierung

Die in dieser Studie verwendeten Plasmide und *S.-cerevisiae-Stämme* sind in den Tabellen 2 und 3 aufgeführt.

**Tabelle 2. Verwendete Plasmide**

| **Plasmid-Bezeichnung** | **Beschreibung** | **Referenz** |
|---|---|---|
| pAG32 | *hphMX* Resi stenzkassette | Euroscarf (SEQ ID NO: 01) |
| pUG66 | *loxP-ble-loxP* DisruptionsKassette | Euroscarf (SEQ ID NO: 02) |
| pUG74 | *loxP-natMX-loxP* DisruptionsKassette | Euroscarf (SEQ ID NO: 03) |
| p41bleTEF-*Opgdh* | *CEN6*/*ARSH4, ble, TEF1p-Opgdh-CYC1t* | Klein et al. (2016) SEQ ID NO: 04 |
| pMA-T_*RofumR* | Codon-optimierte Sequenz für Fumarase *FUM* aus *Rhizopus oryzae* | Thermo Fisher Scientific (SEQ ID NO: 05) |
| pOK-RQ*_TbFRDg-R* | Codon-optimierte Sequenz für Fumaratreduktase *FRD* aus *Trypanosoma brucei* ohne Codons für peroxisomales Targeting | Thermo Fisher Scientific (SEQ ID NO: 06) |
| pUC18*_AnDCT-02 w*/*oSTOP* | Codon-optimierte Sequenz für den Dicarbonsäure-Transporter *DCT-02* aus *Aspergillus niger* | GenScript (SEQ ID NO: 07) |
| pUC18 | *E. coli* Cloning-Vektor | Yanisch-Perron et al. (1985) (SEQ ID NO: 08) |
| pUC18- *P_{ACT1}-DAK1* | P*_{ACT1}-SeDAK1*-T*_{TPS1}* | Klein et al. (2016) (SEQ ID NO: 09) |
| pUC18-P*_{PGK1}-CjFPS1* | P*_{PGK1}-CjFPS1-*T*_{RPL15A}* | Klein et al. (2016) SEQ ID NO: 10 |
| pUC18-P*_{JEN1}-MDH3-R* | P*_{JEN1}-SCMDH-R*-T*_{IDP1}* | Anmeldung (SEQ ID NO: 11) |
| pUC18-P*_{HOR7}-RofumR* | P*_{HOR7}-RofumR*-T*_{DIT1}* | Anmeldung (SEQ ID NO: 12) |
| pUC18-P*_{FBA1}-TbFRDg-R* | P*_{FBA1}-TbFRDg-R-*T*_{ADH1}* | Anmeldung (SEQ ID NO: 13) |
| pUC18-P*_{COX7}-AnDCT-02* | P*_{COX7}-AnDCT-02*-T*_{CYC1}* | Anmeldung (SEQ ID NO: 14) |
| pCfB3045 | 2 µm, *natMX6, SNR52p-gRNA.* XI-3-*SUP4t* | Jessop-Fabre et al. (2016) (SEQ ID NO: 15) |
| *p414-TEF1p-Cas9-CYC1t* | *CEN6*/*ARSH4, TRP1, TEF1p-cas9-CYC1t* | DiCarlo et al. (2013) (SEQ ID NO: 16) |
| p414-*TEF1p-Cas9-CYC1t-nat1* | *CEN6*/*ARSH4, natMX4, TEF1p-cas9-CYC1t* | Klein et al. (2016) (SEQ ID NO: 17) |
| p414-*TEF1p-Cas9-CYC1t-hphMX* | *CEN6*/*ARSH4, hphMX, TEF1p-cas9-CYC1t* | Anmeldung (SEQ ID NO: 18) |
| p426-SNR52p-gRNA.*YGLCt3-SUP4t-hphMX* | 2 µm, *hphMX, SNR52p-gRNA.YGLCτ3-SUP4t* | Islam et al. (2017) (SEQ ID NO: 19) |
| p426-SNR52p-gRNA.*YPRCτ3-SUPt-hphMX* | 2 µm, *hphMX, SNR52p-gRNA.YPRCτ3-SUP4t* | Xiberras et al. (2020) (SEQ ID NO: 20) |

**Tabelle 3. Verwendete Stämme**

| **Stamm bezeichnung** | **Genotyp** | **Referenz** |
|---|---|---|
| CEN.PK113-1A | *MATα* (prototroph) | Euroscarf |
| CEN.PK113-1A *UBR2_{JL1} GUT1_{JL1}* | *ubr2::UBR2_{JL1}; gut1::GUT1_{JL1}* | Ho et al. (2017) |
| CEN.PK113-1A *UBR2_{CBS} GUT1_{JL1}* | *ubr2::UBR2C_{CBS 6412-13A}; gut1::GUT1_{JL1}* | Stamm basiert auf CEN.PK113-1A *GUT1_{CBS} UBR2_{CBS}* (Swinnen et al, 2016), bei dem das Allel *GUT1_{CBS 6412-13A}* ersetzt wurde durch das Allel *GUT1_{JL1},* unter Verwendung des von Ho et al. (2017) für den Allelersatz von *GUT1* beschriebenen Verfahrens. |
| *UBR2*_{CBS}-DHA | *ubr2::UBR2_{CBS 6412-13A}; gut1::loxP-ble-loxP; YGLCτ3::P_{TDH3}-CjFPS1-T_{RPL15A}*-*P_{TEF1}-Opgdh-T_{CYC1}-P_{ADH2}-ScDAK1-T_{TPS1}* | Anmeldung |
| *UBR2*_{CBS}-DHA-SA-*An*DCT-02 | *ubr2::UBR2_{cBS 6412-13A}; gut1::loxP-ble-loxP; YGLCτ3::P_{TDH3}-CjFPS1-T_{RPL15A}*-*P_{TEF1}-Opgdh-T_{CYC1}-P_{ADH2}-ScDAK1-T_{TPS1}; YPRCτ3::P_{JEN1}-ScMDH3-R-T_{IDP1}-P_{HOR7}*-*RofumR-T_{DIT1}-P_{FBA1}-TbFRDg-R-T_{ADH1}*-*P_{COX7}-AnDCT-02-T_{CYC1}* | Anmeldung |
| *UBR2*_{CBS}-DHA-SA-*An*DCT-02-*PYC2*oe | *ubr2::UBR2_{CBS 6412-13A}; gut1::loxP-ble-loxP; YGLCτ3::P_{TDH3}-CjFPS1-T_{RPL15A}*-*P_{TEF1}-Opgdh-T_{CYC1}-P_{ADH2}-ScDAK1-T_{TPS1}; YPRCτ3::P_{JEN1}-ScMDH3-R-T_{IDP1}-P_{HOR7}*-*RofumR-T_{DIT1}-P_{FBA1}-TbFRDg-R-T_{ADH1}*-*P_{COX7}-AnDCT-02-T_{CYC1;} XI-3::P_{HOR7}*-*ScPYC2-T_{TPS1}* | Anmeldung |
| *UBR2*_{CBS}-DHA-SA-*An*DCT*-*02 *icl1Δ* | *ubr2::UBR2_{CBS 6412-13A}; gut1::loxP-ble-loxP; icl1::loxP*-natMX-*loxP; YGLCτ3::P_{TDH3}-CjFPS1-T_{RPL15A}-P_{TEF1}*-*Opgdh-T_{CYC1}-P_{ADH2}-ScDAK1-T_{TPS1}; YPRCτ3::P_{JEN1}-ScMDH3-R-T_{IDP1}-P_{HOR7}*-*RofumR-T_{DIT1}-P_{FBA1}-TbFRDg-R-T_{ADH1}*-*P_{COX7}-AnDCT-02-T_{CYC1}* | Anmeldung |
| *UBR2*_{CBS}-DHA-SA-*An*DCT-02-PYC2oe *icl1Δ* | *ubr2::UBR2_{CBS 6412-13A}; gut1::loxP-ble-loxP; icl1::loxP-*natMX*-loxP; YGLCτ3::P_{TDH3}-CjFPS1-T_{RPL15A-}P_{TEF1}*-*Opgdh-T_{CYC1}-P_{ADH2}-SeDAK1-T_{TPS1}; YPRCτ3::P_{JEN1}-ScMDH3-R-T_{IDP1}*-*P_{HOR7}*-*RofumR-T_{DIT1}-P_{FBA1}-TbFRDg-R-T_{ADH1}*-*P_{COX7}-AnDCT02-T_{CYC;} XI-3::P_{HOR7}*-*ScPYC2-T_{TPS1}* | Anmeldung |
| *UBR2*_{CBS}-DHA-SA-*An*DCT-02 (old) | *gut1::P_{TEF1-}Pagdh-T_{cYC1}; P_{ACT1}-DAK1-T_{TPS1}; P_{PGK1}-PjFPS1-T_{RPL15A}; P_{TDH3}*-*DAK1-T_{IDP1}; ubr2::UBR2_{CBS 6412-13A}; YGLCτ3::P_{PGK1}-MDH3-R-T_{IDP1}; P_{TEF1}*-*RofumR-T_{RPL15A}; P_{TDH3}-TbFRDg-R-T_{CYC1};* YPRCT3*::P_{ENO2}-AnDCT-02-T_{DIT1}* | Xiberras et al. (2020) |

Hefezellen wurden routinemäßig auf festem YPD-Medium angezogen, das 10 g L⁻¹ Hefeextrakt, 20 g L⁻¹ Pepton, 20 g L⁻¹ Glucose und 15 g L⁻¹ Agar enthielt. Agarplatten wurden in einem statischen Inkubator bei 30 °C kultiviert. Medien wurden bei Bedarf zu Selektionszwecken mit Phleomycin (20 mg L⁻¹), Hygromycin B (300 mg L⁻¹) oder Nourseothricin (100 mg L⁻¹) supplementiert. *E. coli* DH5α wurde zur Plasmidkonstruktion und -isolierung verwendet, und die Zellen wurden routinemäßig in Lysogenie-Bouillon (LB) gezüchtet, die 10 g L⁻¹ NaCl, 5 g L⁻¹ Hefeextrakt, 10 g L⁻¹ Pepton enthielt, und auf einen pH von 7,5 mit 2 M NaOH eingestellt (Bertani, 1951). Zur Selektion und Aufrechterhaltung von Plasmid-haltigen Zellen wurden 100 mg L⁻¹ Ampicillin zugegeben. Die Kultivierungen wurden auf einem Orbitalschüttler bei 250 U/min und 37 °C durchgeführt, und Plasmide wurden unter Verwendung des GeneJET^{™} Plasmid-Miniprep-Kits (Thermo Fisher Scientific, Waltham, MA, USA) isoliert.

### Allgemeine molekularbiologische Techniken

Präparative PCRs zur Klonierung und Sequenzbestimmung integrierter Expressionskassetten wurden unter Verwendung von Phusion^{®} High-Fidelity-DNA-Polymerase (New England BioLabs, Frankfurt am Main, Deutschland) durchgeführt. Die PCR-Bedingungen wurden an die Richtlinien des Herstellers angepasst. Restriktionsenzyme, alkalische FastAP-Phosphatase und T4-DNA-Ligase wurden von Thermo Fisher Scientific erhalten und gemäß den Anweisungen des Herstellers verwendet. PCR-Produkte wurden unter Verwendung des GeneJET^{™} PCR-Purification-Kit (Thermo Fisher Scientific) gereinigt, und nach der Restriktion erhaltene DNA-Fragmente wurden herausgeschnitten und unter Verwendung des QIAquick Gel-Purification-Kit (Qiagen, Hilden, Deutschland) gereinigt. Die Transformation von *S. cerevisiae* mit Plasmiden sowie linearen Expressionskassetten zur genomischen Integration wurde gemäß der von Gietz et al. (1995) beschriebenen Lithium-Acetat-Methode durchgeführt.

### Konstruktion der Expressionskassetten für die genomische Integration zur Etablierung des reduktiven Weges für die Bernsteinsäureproduktion (inklusive Transporter)

Die zur Konstruktion der Expressionskassetten verwendeten Plasmide sind in Tabelle 2 aufgeführt. Codon-optimierte Codierungssequenzen für *Rhizopus oryzae fumR* (*RofumR*)*,* und *Trypanosoma brucei FRDg* (*TbFRDg-R*) wurden über die GeneArt^{™}-Gensynthese von Thermo Fisher Scientific erhalten, während die entsprechende Sequenz für *Aspergillus niger DCT-02* ohne das Stopcodon (*AnDCT-02 w*/*oSTOP*) durch die Firma GeneScript synthetisiert wurde. Mdh3 und TbFRDg wurden durch Entfernen des peroxisomalen Targeting-Signals (SKL) aus dem Protein auf das Cytosol ausgerichtet (kodierende Sequenzen: *MDH3-R; TbFRDg-R*)*.* Die Kassetten für *S. cerevisiae MDH3-R* (*ScMDH3-R*) unter der Kontrolle des Promotors **P***_{JEN1}* und des Terminators **T***_{IDP1}, RofumR* unter der Kontrolle des Promotors **P***_{HOR7}* und des Terminators **T***_{DIT1}, TbFRDg-R* unter der Kontrolle des Promotors **P***_{FBA1}* und des Terminators **T***_{ADH1}* und für *AnDCT-02* unter Kontrolle des Promotors **P***_{COX7}* und des Terminators **T***_{CYC1}* wurden in pUC18 unter Verwendung einer isothermen Gibson-Assemblierung zusammengesetzt (Gibson et al., 2009). Alle zur Amplifikation der jeweiligen Promotoren, Codierungssequenzen und Terminatoren für die Gibson-Assemblierung verwendeten Primer sind in Tabelle 4 aufgeführt.

**Tabelle 4. Primer für die Plasmidkonstruktion**

| **Konstruiertes Plasmid** | **Am plifiziertes Fragment** | **Template** | **Primernummer** | **Sequenz** |
|---|---|---|---|---|
| *p414-TEF1p-Cas9-CYC1t-hphMX* | hphMX6 | pAG32 | 445 | TTACAATTTCCTGATGCGGT ATTTTCTCCTTACGCATCTG TGCGGTATTTCACACCGCAT CCAGCTGAAGCTTCGTACG C (SEQ ID NO: 21) |
| | | | 446 | GCGCGTTTCGGTGATGACG GTGAAAACCTCTGACACAT GCAGCTCCCGGAGACGGTC ACAGCATAGGCCACTAGTG GATCTG (SEQ ID NO: 22) |
| pUC18-*MDH3* | Linearisierter pUC18 Backbone | pUC18 | 1364 | AAGTTCGGATCATTACCATC GTAATCATGGTCATAGCTGT TTCCT (SEQ ID NO: 23) |
| | | | 1365 | ACGGCATCTAATCGTTCTAA GGCACTGGCCGTCG (SEQ ID NO: 24) |
| | P*_{JEN1}* | CEN.PK1 13-7D *UBR2_{JL1} GUT1_{JL1}* genomic DNA | 1366 | GTAAAACGACGGCCAGTGC CTTAGAACGATTAGATGCC GTCAATATGTATGTGC (SEQ ID NO: 25) |
| | | | 1367 | AGAATTGCGACTTTGACCAT ATTTTCAGTAGCAGTATTTA ATCTCTTTGAGGAAAAACT (SEQ ID NO: 26) |
| | *ScMDH3-R* | CEN.PK1 13-7D *UBR2_{JL1} GUT1_{JL1}* genomic DNA | 1368 | TAAATACTGCTACTGAAAA TATGGTCAAAGTCGCAATTC TTGGC (SEQ ID NO: 27) |
| | | | 432 | ACGTAAATTCGATCAAGAG TCTAGGATGAAACTCTTGCC (SEQ ID NO: 28) |
| | *T_{IDP1}* | CEN.PK1 13-7D *UBR2_{JL1} GUT1_{JL1}* genomic DNA | 431 | CTAGACTCTTGATCGAATTT ACGTAGCCCAATCTACCAC (SEQ ID NO: 29) |
| | | | 1369 | ACAGCTATGACCATGATTA CGATGGTAATGATCCGAAC TTGGGGA (SEQ ID NO: 30) |
| pUC18-*RofumR* | Linearisierter pUC18 Backbone | pUC18 | 1370 | AGAAAAGCGTTGCGGAGTA AGTAATCATGGTCATAGCT GTTTCCTGTGT (SEQ ID NO: 31) |
| | | | 1371 | GAGTGTCTCCGATGGCAAA GGGCACTGGCCGTCG |
| | | | | (SEQ ID NO: 32) |
| | P*_{HOR7}* | CEN.PK1 13-7D *UBR2_{JL1} GUT1_{JL1}* genomic DNA | 1372 | GTAAAACGACGGCCAGTGC CCTTTGCCATCGGAGACACT CTG (SEQ ID NO: 33) |
| | | | 1373 | AACCTTGGAGAGTTGTTCAT TTTTTATTATTAGTCTTTTTT TTTTGACAATATTTGTATGA TTTGATAGT (SEQ ID NO: 34) |
| | *RofumR* | pMA-T_ RofumR | 1374 | AAAAAGACTAATAATAAAA AATGAACAACTCTCCAAGG TTGTTCTC (SEQ ID NO: 35) |
| | | | 1375 | AATGTAGCGCTCTTACTTTA TTAGTCCTTAGCAGAAATCA TATCTTCTGGTCTGACC (SEQ ID NO: 36) |
| | T*_{DIT1}* | CEN.PK1 13-7D *UBR2_{JL1} GUT1_{JL1}* genomic DNA | 1376 | TGATTTCTGCTAAGGACTAA TAAAGTAAGAGCGCTACAT TGGTCTACCT (SEQ ID NO: 37) |
| | | | 1377 | ACAGCTATGACCATGATTA CTTACTCCGCAACGCTTTTC TGAACG (SEQ ID NO: 38) |
| pUC18-*TbFRDg-R* | Linearisierter pUC18 Backbone | pUC18 | 1378 | ATTGACCACACCTCTACCGG GTAATCATGGTCATAGCTGT TTCCTGTGTG (SEQ ID NO: 39) |
| | | | 1379 | CCACACATTTTTGAGATTTT GGCACTGGCCGTCG (SEQ ID NO: 40) |
| | P*_{FBA1}* | CEN.PK1 13-7D *UBR2_{JL1} GUT1_{JL1}* genomic DNA | 1380 | GTAAAACGACGGCCAGTGC CAAAATCTCAAAAATGTGT GGGTCATTACG (SEQ ID NO: 41) |
| | | | 1381 | GAAGATCTACCGTCAACCA TTTTGAATATGTATTACTTG GTTATGGTTATATATGACAA AAGA (SEQ ID NO: 42) |
| | *TbFRDg-R* | pMA-T_ TbFRDg-R | 1382 | CCAAGTAATACATATTCAA AATGGTTGACGGTAGATCTT CTGC (SEQ ID NO: 43) |
| | | | 1383 | ATAAATCATAAGAAATTCG CTTAAGAACCTGATGGTTCG GTTTCGTC (SEQ ID NO: 44) |
| | T*_{ADH1}* | CEN.PK1 13-7D | 1384 | CCGAACCATCAGGTTCTTAA GCGAATTTCTTATGATTTAT |
| | | *UBR2_{JL1} GUT1_{JL1}* genomic DNA | | GATTTTTATTATTAAATAAG TTATAAAA (SEQ ID NO: 45) |
| | | | 1385 | ACAGCTATGACCATGATTA CCCGGTAGAGGTGTGGTCA ATAAGAG (SEQ ID NO: 46) |
| pUC18-P*_{COX7}*-*AnDCT-02* | Linearisierter pUC18 Backbone | pUC18 | 1386 | GCTCGAAGGCTTTAATTTGC GTAATCATGGTCATAGCTGT TTCCT (SEQ ID NO: 47) |
| | | | 1387 | GAAGGTAAATAACTGAAAA GGGCACTGGCCGTCG (SEQ ID NO: 48) |
| | P*_{COX7}* | CEN.PK1 13-7D *UBR2_{JL1} GUT1_{JL1}* genomic DNA | 1388 | GTAAAACGACGGCCAGTGC CCTTTTCAGTTATTTACCTT CCTTTCTCTCACG (SEQ ID NO: 49) |
| | | | 1389 | AAGGATGTTTCTACGTTCAT TATTTATTATTGTTTTGCTTT CTGTACTTGTTGGTTTAGA (SEQ ID NO: 50) |
| | *AnDCT-02* | pUC18_ *AnDCT02 w*/*oSTOP* | 1390 | AAGCAAAACAATAATAAAT AATGAACGTAGAAACATCC TTGCCAG (SEQ ID NO: 51) |
| | | | 1391 | AATGTAAGCGTGACATAAC TAATTACATGATTATTCGGA GACATCTTCATCTTGACCAG (SEQ ID NO: 52) |
| | *T_{CYC1}* | CEN.PK1 13-7D *UBR2_{JL1} GUT1_{JL1}* genomic DNA | 1392 | ATGAAGATGTCTCCGAATA ATCATGTAATTAGTTATGTC ACGCTTACATTCAC (SEQ ID NO: 53) |
| | | | 1393 | ACAGCTATGACCATGATTA CGCAAATTAAAGCCTTCGA GCGT (SEQ ID NO: 54) |

Die codierenden Sequenzen von *RofumR, TbFRDg-R* und *An*DCT-02 wurden aus den Plasmiden pMA-T_RofumR, pMA-T_TbFRD bzw. pUC18_AnDCT-02 w/oSTOP amplifiziert (Tabelle 2). Die codierende Sequenz von *ScMDH3* und alle Promotoren und Terminatoren wurden aus genomischer DNA amplifiziert, die aus dem *S. cerevisiae-*Stamm CEN.PK113-7D *UBR2_{JL1} GUT1_{JL1}* isoliert wurde (Ho et al., 2017). Einstufige isotherme DNA-Assemblierungsreaktionen wurden wie von Gibson et al. (2009) beschrieben vorgenommen und enthielten 15 µl der Reagenz-Enzymmischung, 0,05 pmol *Bam*HI-linearisiertes pUC18 und einen dreifachen Überschuss der Inserts (Promotor, codierende Sequenz und Terminator (jeweils 0,15 pmol)) in einem Endvolumen von 20 µl. Reaktionsmischungen wurden 1 h bei 50 °C inkubiert. *E. coli* DH5α-Zellen wurden mit 5 µL der Reaktion und den resultierenden Vektoren mit den Bezeichnungen pUC18-*MDH3-*R, *pUC18-RofumR,* pUC18-TbFRDg-R und pUC18-*AnDCT-02 w*/*oSTOP* transformiert (Tabelle 2).

### Plasmide für die CRISPR-Cas9-vermittelte Genom-Editierung bei S. cerevisiae

Für die CRISPR-Cas9-vermittelte Genom-Editierung wurden sowohl Cas9 als auch die die Endonuklease zur Integrationsstelle führende gRNA aus Plasmiden exprimiert (Tabelle 2). Das Plasmid p414*-TEF1p-Cas9-CYC1t-hphMX* wurde in Analogie zu p414-*TEF1p-Cas9-CYC1t-nat1* konstruiert (Klein et al., 2016). Das Plasmid p414-*TEF1p-Cas9-CYC1t* wurde als Ausgangspunkt verwendet und war ein Geschenk von George Church (Addgene-Plasmide # 43802). Der auxotrophe *TRP1*-Marker wurde bei *S. cerevisiae* CEN.PK113-1A mittels homologer Rekombination *in vivo* durch eine Expressionskassette ersetzt, die Resistenz gegen Hygromycin B (in p414-*TEF1p-Cas9-CYC1t*) verleiht. Die Markerkassette wurde aus pAG32 (Goldstein und McCusker, 1999) unter Verwendung der Primer 445 und 446 (Tabelle 4) amplifiziert. Die Primer enthielten 5'-terminale Sequenzen von 60 bp, die homolog zu Regionen stromaufwärts und stromabwärts der *TRP1-*Markerkassetten in p414-*TEF1p-Cas9-CYC1t* waren. Der Vektor wurde innerhalb der TRP1-Codierungssequenz unter Verwendung von *Mun*I linearisiert. CEN.PK113-1A wurde dann co-transformiert mit dem linearisierten Vektor und dem PCR-amplifizierten Resistenzmarker zur Assemblierung mittels homologer Rekombination *in vivo,* und der resultierende Vektor wurde als p414-*TEF1p-Cas9-CYC1t-hphMX* bezeichnet.

### S.-cerevisiae-Stammkonstruktion

### Allgemeine Strategien für genomische Integrationen über CRISPR-Cas9

Für genomische Integrationen über CRISPR-Cas9 wurden die "long terminal repeats" YGLCτ3 und YPRCτ3 auf den Chromosomen VII bzw. XVI (Bai Flagfeldt et al., 2009) und Position XI-3 (Jessop-Fabre et al., 2016) verwendet. Um Cas9 auf die oben genannten genomischen Stellen auszurichten, wurden die Vektoren p426-*SNR52p-gRNA.YGLCt3-SUP4t-hphMX,* p426-*SNR52p-gRNA.YPRCt3-SUP4t-hphMX* und pCfB3045 verwendet, die Expressionskassetten für die jeweiligen gRNAs tragen (Tabelle 2). Entsprechend dem verwendeten Resistenzmarker für die gRNA-Expression wurde Cas9 entweder unter Verwendung des Plasmids p414-*TEF1p-Cas9-CYC1t-nat1* oder p414-*TEF1p-Cas9-CYC1t-hphMX* exprimiert (Tabelle 2). DNA-Fragmente zur Assemblierung und Integration (entweder ganze Expressionskassetten oder Teilen davon) wurden aus den in Tabelle 2 aufgeführten Vektoren oder aus genomischer DNA, die aus dem Stamm CEN.PK113-1A *UBR2_{JL1} GUT1_{JL1}* isoliert wurde, PCR-amplifiziert (Ho et al., 2017). Die verwendeten Primer (Tabelle 5) enthielten 5'-Verlängerungen, die 40-60 bp-Sequenzen erzeugten, die zu Regionen direkt stromaufwärts und stromabwärts des inserierten Doppelstrangbruchs an der Integrationsstelle oder zum jeweiligen benachbarten Fragment homolog waren (falls mehrere Kassetten an demselben Ort assembliert wurden). Co-Transformation des *S*. *cerevisiae*-Stammes*,* der die Cas9-Endonuklease exprimiert, mit äquimolaren Mengen der Expressionskassetten und des jeweiligen Vektors für die gRNA-Expression führte zur Assemblierung und Integration aller Expressionskassetten am Zielort. Positive Transformanten wurden auf YPD-Agar selektiert, der sowohl Nourseothricin als auch Hygromycin B enthielt. Beide Vektoren wurden anschließend durch serielle Transfers in YPD-Medium, dem die jeweiligen Antibiotika fehlten, aus dem resultierenden Klon entfernt, was den gewünschten Stamm ergab. Anschließend wurden alle integrierten Expressionskassetten sequenziert.

**Tabelle 5. Verwendete Primer und Templates für die Amplifikation von Disruptions- und Intergrationskassetten**

| **Konstrukt** | *Integra-* Amplifiziertes *tionsort* Fragment | | Template | Primer nummer | Sequenz |
|---|---|---|---|---|---|
| **gut1::loxP-ble-loxP** | *GUT1* / *YHL032C* | loxP-PTEF1-ble-TTEF1-loxP | pUG66 | 111 | TGTGGGGGGATGCCTGTTCTCGAACCATATA AAATATACCATGTGCAGCTGAAGCTTCGTAC GC |
| | | | | 476 | AAGGTGGAGAGGAATATAAAATTATGGAAAT TACATTGTTAATAGAAATTATTTATGTTGGCA TAGGCCACTAGTGGATCTG |
| **DHA-Modul** | *YGLCτ3* (Bai Flagfeldt et al., 2009) | *P_{TEF1}-Opgdh-T_{CYC1}* | p41bleTEF-Opgdh | 1272 | AGTTTTTCCGTCTCTGG CTGAAGGCTCATTTCCATGATGGGGTCACAA TTATTATCGCACATAGCTTCAAAATGTTTCTA CTC |
| | | | | 986 | GCCATTGGTATATCTAGACCTGGTAATGGTG CAAATTAAAGCCTTCGAGC |
| | | P*_{ADH2}* | CEN.PK11 3-7D *UBR2_{JL1} GUT1_{JL1}* genomic DNA | 984 | GTTTTGGGACGCTCGAAGGCTTTAATTTGCAC CATTACCAGGTCTAGATATACC |
| | | | | 985 | CTTCAAACGATTTAGCGGACATTGTGTATTAC GATATAGTTAATAGTTGATAG |
| | | *ScDAK1-*T*_{TPS1}* | pUC18-*P_{ACT1}-DAK1* | 983 | TATCAACTATTAACTATATCGTAATACACAAT GTCCGCTAAATCGTTTGAAG |
| | | | | 1273 | GTATTCTTTGAAATGGCAGTATTGATAATGA GATAAATTATGGCTTTTTTTCTTGATTTCC |
| **DHA-Modul** | *YGLCτ3* (Bai Flagfeldt et al., 2009) | *P_{TDH3}* | CEN.PK11 3-7D *UBR2_{JL1} GUT1_{JL1}* genomic DNA | 1275 | GAAAAAGGAAATCAAGAAAAAAAAGCCATA ATTTATCTCATTATCAATACTGCCATTTC |
| | | | | 1276 | GAACTACAGCCTTCACCACTAGCAAGTAATT CTCCTGTCATTTTGTTTGTTTATGTGTGTTTAT TCG |
| | | *CjFPS1*-T*_{RLP15A}* | pUC18-*P_{PGK1}*-*CjFPS1* | 1277 | CAAGAACTTAGTTTCGAATAAACACACATAA ACAAACAAAATGACAGGAGAATTACTTGCTA G |
| | | | | 1278 | ATGTAGTTCAACTTCTATGAATGCTCGGCGAT ACGATATGGGAAAAACGGGAAGAAAAGG |
| | | | | | |
| **SA-Modul + AnDCT-02 Transporter SA-Modul + AnDCT-02 Transporter** | *YPRCτ3* (Bai Flagfeldt et al., 2009) *YPRCτ3* (Bai Flagfeldt et al., 2009) | P*_{JEN1}-ScMDH3-R-*T*_{IDP1}* | pUC18-*MDH3-R* | 1088 | GTATTTTTAATCGTCCTTGTATGGAAGTATCA AAGGGGACGTTCTTCACCTCCTTGGAATTAG AACGATTAGATGCCGTC |
| | | | | 1395 | GCCTCGGTCAGAGTGTCTCCGATGGCAAAGG ATGGTAATGATCCGAACTTG |
| | | P*_{HOR7}-RofumR-*T*_{DIT1}* | pUC18-*RofumR* | 1396 | AAGGTTCCCCAAGTTCGGATCATTA CCATCCTTTGCCATCGGAGACAC |
| | | | | 1398 | ACGTAATGACCCACACATTTTTGAGATTTTTT ACTCCGCAACGCTTTTC |
| | | P*_{FBA1}-TbFRDg-R-T_{ADH1}* | pUC18-*TbFRDg-R* | 1399 | GCGGGCGTTCAGAAAAGCGTTGCGGAGTAAA AAATCTCAAAAATGTGTGGG |
| | | | | 1401 | TGAGAGAAAGGAAGGTAAATAACTGAAAAG CCGGTAGAGGTGTGGT |
| **SA-Modul + AnDCT -02 Transporter** | *YPRCτ3* (Bai Flagfeldt et al., 2009) | P*_{COX7}-AnDCT-02-*T*_{CYC1}* | pUC18-*AnDCT-02 w*/*oSTOP* | 1402 | GGTCGCTCTTATTGACCACACCTCTACCGGCT TTTCAGTTATTTACCTTCCTTT |
| | | | | 774 | GATTAAATAAAGAATGATTTACAATCTAGTC GCAAAAACAAGTACAGTGCTGACGTCCCATC GCAAATTAAAGCCTTCGAG |
| | | | | | |
| **PYC2-Über-expressionsKassette** | XI-3 (Jessop Fabre et al., 2016) | P*_{HOR7}* | CEN.PK11 3-7D *UBR2_{JL1} GUT1_{JL1}* genomic DNA | 1518 | GACACATCTAACTGATTAGTTTTCCGTTTTAG GATATTGACGCTTTGCCATCGGAGACACTCT GA |
| | | | | 1519 | TCCCTAAGACCGGCCAATTTCTTGCTACTGCT CATTTTTTATTATTAGTCTTTTTTTTTTGACAA TATTTGTATG |
| | | *ScPYC2* | CEN.PK11 3-7D *UBR2_{JL1} GUT1_{JL1}* genomic DNA | 1520 | ATACAAATATTGTCAAAAAAAAAAGACTAAT AATAAAAAATGAGCAGTAGCAAGAAATTGG |
| | | | | 1521 | CGGACCAGGAATAGACGATCGTCTCATTTGC ATCGGGTTCATTACTTTTTTGGGATGGGGGT A |
| | | T*_{TPS1}* | CEN.PK11 3-7D *UBR2_{JL1} GUT1_{JL1}* genomic DNA | 1522 | GTCCTAGAAGAAGAAACCCTACCCCCATCCC AAAAAAAGTAATGAACCCGATGCAAATGAG AC |
| | | | | 1523 | GTATTGCTGGCTCAATCCACGTAAGGAAAAT GATCAGCCCGATAAATTATGGCTTTTTTTCT TGATTTCC |
| **PYC2-Über-expressionsKassette** | XI-3 (Jessop Fabre et al., 2016) | P*_{HOR7}* | CEN.PK11 3-7D *UBR2_{JL1} GUT1_{JL1}* genomic DNA | 1518 | GACACATCTAACTGATTAGTTTTCCGTTTTAG GATATTGACGCTTTGCCATCGGAGACACTCT GA |
| | | | | 1519 | TCCCTAAGACCGGCCAATTTCTTGCTACTGCT CATTTTTTATTATTAGTCTTTTTTTTTTGACAA TATTTGTATG |
| | | *ScPYC2* | CEN.PK11 3-7D *UBR2_{JL1} GUT1_{JL1}* genomic DNA | 1520 | ATACAAATATTGTCAAAAAAAAAAAGACTAAT AATAAAAAATGAGCAGTAGCAAGAAATTGG |
| | | | | 1521 | CGGACCAGGAATAGACGATCGTCTCATTTGC ATCGGGTTCATTACTTTTTTGGGATGGGGGT A |
| | | T*_{TPS1}* | CEN.PK11 3-7D *UBR2_{JL1} GUT1_{JL1}* genomic DNA | 1522 | GTCCTAGAAGAAGAAACCCTACCCCCATCCC AAAAAAAGTAATGAACCCGATGCAAATGAG AC |
| | | | | 1523 | GTATTGCTGGCTCAATCCACGTAAGGAAAAT GATCAGCCCGATAAATTATGGCTTTTTTTCT TGATTTCC |
| | | | | | |
| **icl1::loxP-natMX-loxP** | *ICL1* / YER065C | loxP-P_{TEF1}-natMX-T_{TEF1}-loxP | pUG74 | 1189 | AACAATTGAGAGAAAACTCTTAGCATAACAT AACAAAAAGTCAACGAAAACCAGCTGAAGC TTCGTACGC |
| | | | | 1190 | ATATACTTGTCAGGAAATGCCGGCAGTTCTA ATGGTTAATCCTTGTCCGCATAGGCCACTAGT GGATCTG |

### Gendeletionen

Die Deletionen von *GUT1* und *ICL1* wurden unter Verwendung von Disruptions-Kassetten erhalten, die aus dem Phleomycin-Resistenzmarker (*ble*) und dem Clonat-Resistenzmarker (*natMX*) bestanden. Die Disruptions-Kassetten wurden aus den Plasmiden pUG66 und pUG74 (Tabelle 2) unter Verwendung der Primerpaare 111/476 bzw. 1189/1190 (Tabelle 5) amplifiziert. Die zur Amplifikation verwendeten Primer enthielten an ihrem 5'-terminalen Ende eine 60-nt-Sequenz, die zu der Region unmittelbar stromaufwärts oder stromabwärts von dem Start- oder Stoppcodon des zu deletierenden Gens komplementär ist. Nach der PCR-Amplifikation und Reinigung wurden die Disruptions-Kassetten zur Transformation der jeweiligen *S.-cerevisiae*-Stämme verwendet.

### Rekonstruktion des Stammes UBR2_{CBS}-DHA-SA-AnDCT-02

Der Stamm CEN.PK113-1A *UBR2_{CBS} GUT1_{JL1}* (Mat alpha), der zuvor auf der Basis des *S. cerevisiae* Stammes CEN.PK113-1A durch Ersetzen der endogenen *UBR2-* und *GUT1-*Allele durch jene von CBS 6412-13A (*UBR2_{CBS 6412-13A}*) und CEN.PK113-7D *JL1* (*GUT1_{JL1}*) konstruiert worden war (Details in Tabelle 3), diente als Ausgangspunkt für die Konstruktion des erfindungsgemäßen Stammes. Dieser Ausgangsstamm wurde innerhalb der Studie von Ho et al. (2017) konstruiert, wobei die Allelaustausche exakt in der von den Autoren beschriebenen Weise vorgenommen wurden. Um den erfindungsgemäßen Stamm zu erzeugen, wurde zunächst der Glycerolkatabolismus modifiziert, indem ein DHA-Modul (final bestehend aus den drei Expressionskassetten für *Opgdh, DAK1* und *CjFPS1*) mittels CRISPR-Cas9 in diesen Stamm integriert wurde. Zu diesem Zweck wurden unter Verwendung in Tabelle 4 aufgelisteten DNA-Templates und passenden Primern fünf PCR-Fragmente erzeugt (in der Reihenfolge der Integration: die *Opgdh*-Expressionskassette, der Promotor **P***_{ADH2},* ein Fragment bestehend aus der *DAK1*-Codierungssequenz und dem Terminator **T***_{TPS1},* der Promotor **P***_{TDH3}* und ein Fragment bestehend aus der *CjFPS1-*Codierungssequenz und dem Terminator T*_{RPL15A}*). Die gereinigten PCR-Produkte wurden dann am *YGLCτ3*-Lokus des Stammes CEN.PK113-1A *UBR2_{CBS} GUT1_{JL1}* über CRISPR-Cas9 wie oben beschrieben integriert. Anschließend wurde das *GUT1*-Alle1 unter Verwendung der Phleomycin-Disruptionskassette deletiert. Der resultierende Stamm wurde dann zur Integration eines modifierten SA-Moduls und einer modifizierten Expressionskassette für den Dicarbonsäuretransporter verwendet. Die Expressionskassetten für *ScMDH3-R, RofumR, TbFRDg-R* und *AnDCT-02* wurden aus den zuvor assemblierten Plasmiden pUC18-*MDH3-R,* pUC18*-RofumR,* pUC18-*TbFRDg-R* und pUC18*-AnDCT-02* (Tabelle 2) unter Verwendung der in Tabelle 5 aufgeführten Primerpaare amplifiziert. Diese Kassetten wurden anschließend am *YPRCτ3*-Lokus des Stammes *UBR2*_{CBS}-DHA unter Verwendung des CRISPR-Cas9-Systems wie oben beschrieben integriert, um den rekonstruierten Stamm *UBR2_{CBS}*-DHA-SA-*An*DCT-02 zu erhalten. Der Stamm wurde am 10.01.2021 unter der Zugriffsnummer 58096 bei der belgisch koordinierten Sammlung von Mikroorganismen (Belgian Coordinated Collections of Microorganisms, BCCM) nach dem Budapester Vertrag über die internationale Anerkennung der Hinterlegung von Mikroorganismen für die Zwecke von Patentverfahren hinterlegt.

### Stamm UBR2_{CBS}-DHA-SA-AnDCT-02-PYC2oe

Die Kassette zur Überexpression von endogenem *PYC2* (unter der Kontrolle des Promotors P*_{HOR7}* und des Terminators T*_{TPS1}*) wurde assembliert und unter Verwendung von CRISPR-Cas9 an Position XI-3 in den rekonstruierten Stamm *UBR2_{CBS}*-DHA-SA-*An*DCT-02 integriert. Promotor, codierende Sequenz und Terminator wurden aus genomischer DNA des Stammes CEN.PK113-1A *UBR2_{JL1} GUT1_{JL1}* unter Verwendung der in Tabelle 5 aufgeführten Primer amplifiziert. CRISPR-Cas9-vermittelte Assemblierung und Integration führten zum Stamm *UBR2_{CBS}*-DHA-SA-*An*DCT-02-*PYC2*ₒₑ*.*

### Isolierung genomischer DNA aus S.-cerevisiae-Transformanten und diagnostische PCR

Die korrekte Integration aller Expressions- und Disruptionskassetten wurde durch diagnostische PCR unter Verwendung von OneTaq Quick-Load-DNA-Polymerase und Puffer gemäß den Empfehlungen des Herstellers (New England Biolabs) überprüft. Genomische DNA wurde gemäß einem modifizierten Protokoll von Hoffman und Winston (1987) isoliert. Ungefähr 50 mg Zellen wurden in 200 ml TE-Puffer (10 mM Tris, 1 mM EDTA, pH 8,0) suspendiert. Anschließend wurden 300 mg säuregewaschene Glasperlen (Durchmesser 0,425-0,6 mm) und 200 µl Phenol:Chloroform:Isoamylalkohol (25:24:1) zugegeben. Die Reaktionsbehälter wurden 2 min bei maximaler Geschwindigkeit verwirbelt und 10 min bei 15700 g zentrifugiert. Die wässrige Phase (1 µL) wurde als Template in 25 µL PCR-Reaktionen verwendet. PCR-Primer für die Kontrolle der korrekten genomischen Integration wurden so gestaltet, dass sie stromaufwärts und stromabwärts der anvisierten genomischen Integrationsstellen sowie innerhalb der integrierten Expressions-/Deletionskassette binden. Zur Analyse der Integration mehrerer Expressionskassetten wurden zusätzliche Primer entwickelt, um Amplikons zu erzeugen, die die Verbindungsstellen zwischen den einzelnen integrierten Expressionskassetten abdecken.

### Medien und Kultivierungsbedingungen für die Herstellung von SA (Succinat) aus Glycerol

Alle Vor- und Zwischenkulturen wurden in synthetischem Medium kultiviert, das 20 g L⁻¹ Glucose und Ammoniumsulfat als Kohlenstoff- bzw. Stickstoffquelle enthielt. Alle Experimente zur Beurteilung der SA-Produktion bei der Schüttelkolben-Batch-Kultivierung wurden in synthetischem Medium durchgeführt, das 60 ml L⁻¹ (75,6 g L⁻¹) Glycerol als einzige Kohlenstoffquelle mit Harnstoff als Stickstoffquelle enthielt. Das synthetische Medium wurde gemäß Verduyn et al. (1992) hergestellt und enthielt 3 g L⁻¹ KH₂PO₄, 0,5 g L⁻¹ MgSO₄.7H₂O, 15 mg L⁻¹ EDTA, 4,5 mg L⁻¹ ZnSO₄.7H₂O, 0,84 mg L⁻¹ MnCl₂.2H₂O, 0,3 mg L⁻¹ CoCl₂.6H₂O, 0.3 mg L⁻¹ CuSO₄.5H₂O, 0.4 mg L⁻¹ NaMoO₄.2H₂O, 4.5 mg L⁻¹ CaCl₂.2H₂O, 3 mg L-1 FeSO₄.7H₂O, 1 mg L⁻¹ H₃BO₃ und 0.1 mg L-1 KI. Nach der Hitzesterilisation des Mediums wurden filtersterilisierte Vitamine zugegeben. Die endgültigen Vitaminkonzentrationen waren: 0,05 mg L⁻¹ D-(+)-Biotin, 1 mg L⁻¹ D-Pantothensäure-Hemicalciumsalz, 1 mg L⁻¹ Nikotinsäure, 25 mg L⁻¹ Myoinosit, 1 mg L⁻¹ Thiaminchloridhydrochlorid, 1 mg L⁻¹ Pyridoxinhydrochlorid und 0,2 mg L⁻¹ 4-Aminobenzoesäure. Falls Harnstoff als Stickstoffquelle verwendet wurde (in Hauptkulturmedien), wurde nach dem Autoklavieren ein geeignetes Aliquot einer Stammlösung zugegeben, um eine Endkonzentration von 2,8 g L⁻¹ zu erhalten, während in allen anderen Kulturen 5 g L⁻¹ Ammoniumsulfat (in Vorkulturmedien) vor der Hitzesterilisation zugegeben wurde. Der pH-Wert des synthetischen Glucosemediums wurde mit 4 M KOH auf 6,5 eingestellt, während der des synthetischen Glycerinmediums mit 2 M H₃PO₄ auf 4,0 eingestellt wurde. Zur Vorkultivierung wurden Zellen aus einer einzelnen Kolonie verwendet, um 3 ml des synthetischen Glucosemediums in ein 10 ml Glasröhrchen zu inokulieren. Die Kultur wurde anschließend über Nacht unter Orbitalschütteln von 200 U/min und 30 °C inkubiert. Die Vorkultur wurde verwendet, um 10 ml des gleichen Mediums in einen 100 ml Erlenmeyerkolben (verschlossen mit einer Metallkappe) zu inokulieren, wobei eine OD₆₀₀ von 0,2 eingestellt wurde. Diese Kultur, im Folgenden als Zwischenkultur bezeichnet, wurde unter den gleichen Bedingungen 24 Stunden lang kultiviert. Das geeignete Kulturvolumen aus der Zwischenkultur (um später eine OD₆₀₀ von 0,2 in 100 ml synthetischem Glycerinmedium einzustellen) wurde 5 min bei 800 g zentrifugiert und der Überstand verworfen. Das Zellpellet wurde dann einmal gewaschen, indem die Zellen in synthetischem Glycerolmedium resuspendiert wurden. Die Zellsuspension wurde erneut zentrifugiert und in 100 ml des gleichen Mediums in einem 500 ml Erlenmeyerkolben (mit einem Baumwollstopfen verschlossen) resuspendiert, wobei eine endgültige OD₆₀₀ von 0,2 eingestellt wurde. Die Hauptkulturen wurden unter Orbitalschütteln von 200 U/min und 30 °C inkubiert und in regelmäßigen Zeitintervallen wurden Proben zur OD₆₀₀-Bestimmung und HPLC-Analyse entnommen.

Bei Kulturen, denen CaCO₃ zugegeben wurde, wurden 3 g CaCO₃ in einen 500-ml-Schüttelkolben überführt und autoklaviert. Der pH-Wert des synthetischen Glycerolmediums, das für diese Kulturen verwendet wurde, wurde mit 4 M KOH auf 6,0 eingestellt. Hauptkulturen (100 ml) mit einer anfänglichen OD₆₀₀ von 0,2 wurden wie oben beschrieben hergestellt und anschließend wurden die gesamten Zellsuspensionen unter sterilen Bedingungen in die Schüttelkolben gegeben, die das CaCO₃ enthielten. Für OD₆₀₀-Messungen wurden die Proben in 0,2 M HCl verdünnt, um eine vollständige Auflösung des suspendierten CaCO₃ sicherzustellen.

### Metabolitenanalyse durch HPLC

Proben von Kulturüberständen (1 ml) wurden zuerst durch 0,2 mm Minisart RC-Membranfilter (Sartorius, Göttingen, Deutschland) filtriert und erforderlichenfalls bis zur Analyse bei -20°C gelagert. Die Konzentrationen von SA (Bernsteinsäure), Glycerol und Ethanol in Kulturmedien wurden unter Verwendung eines Waters HPLC-Systems (Eschborn, Deutschland) bestimmt, bestehend aus einem binären Pumpensystem (Waters 1525), einem Injektorsystem (Waters 2707) dem Waters-Säulenheizungsmodul WAT038040, einem Refraktionsindex(RI)-Detektor (Waters 2414) und einem Absorptionsdetektor mit zwei Wellenlängen (Waters 2487). Die Proben wurden auf eine Aminex HPX-87H-Kationenaustauschsäule injiziert (Biorad, München, Deutschland), die an eine an eine Micro-guardR-Säule (Biorad) gekoppelt war. Die Eluierung erfolgte mit 5 mM H₂SO₄ als mobiler Phase bei einer Flussrate von 0,6 mL min⁻¹ und einer Säulentemperatur von 45°C. Zur Injektion wurden Volumina von 20 µL Probe verwendet. SA wurde unter Verwendung des Doppelwellenlängen-Absorptionsdetektors (Waters 2487) nachgewiesen, während Ethanol und Glycerol mit dem RI-Detektor (Waters 2414) analysiert wurden. Die Retentionszeit für SA betrug 11,2 min, für Glycerol 13,5 min und für Ethanol 22,7 min. Die Daten wurden mit der Breeze-2-Software (Waters) verarbeitet und analysiert.

### Bioreaktor-Experimente

Für jedes Bioreaktor-Experiment wurden Zellen aus einer einzelnen Kolonie verwendet, um 100 ml synthetisches Medium zu inokulieren, das 75,6 g L⁻¹ Glycerol enthielt (hergestellt wie oben im Abschnitt "Medien und Kultivierungsbedingungen für die Produktion von SA aus Glycerol" beschrieben, aber unter Verwendung von Ammoniumsulfat anstelle von Harnstoff als Stickstoffquelle) in 500-ml-Rundboden-Schüttelkolben gegeben und in einem Innova-44-Inkubator (New Brunswick Scientific, Edison, NJ, USA) bei 30 °C und 200 U/min über Nacht inkubiert. Zellen, die in frischem Medium exponentiell wuchsen, wurden dann verwendet, um die Bioreaktoren bei einer anfänglichen OD von 1,0 (~0,7 g_{CDW} L⁻¹) zu inokulieren. Aerobe Batchkulturen wurden in 2-1-Bioreaktoren (Applikon, Delft, Niederlande) bei einem anfänglichen Arbeitsvolumen von 1 L synthetischem Glycerolmedium mit Ammoniumsulfat (5 g L⁻¹) als Stickstoffquelle herangezogen. Um höhere Biomassekonzentrationen aufrechtzuerhalten, wurden die Vitamin- und Spurenelementkonzentrationen (zweifach) erhöht und zusätzliches Biotin wurde separat zugegeben, um eine mittlere Endkonzentration von 1 mg L ⁻¹ zu erreichen, wie von Wahl et al. beschrieben. (2017). Der pH-Wert der Kultur wurde durch automatische Zugabe von 2 M KOH bei 5,0 gehalten, und die Temperatur wurde bei 30°C gehalten. Das einströmende Gas war eine Inline-Mischung aus Druckluft und reinem CO₂ (>99,7 % Reinheit, Linde Gas Benelux, Schiedam, Niederlande) mit einer kombinierten Flussrate von 500 mL min⁻¹, gesteuert mit Massendurchflussreglern (Brooks, Hatfield, PA, Vereinigte Staaten). Das resultierende Gasgemisch sollte 10 % CO₂ und 19 % Sauerstoff enthalten, und wurde unter Verwendung eines NGA-2000-Analysators (Rosemount Analytical, Orrville, OH) kontinuierlich gemessen. Der Bioreaktor wurde mit 800 U/min gerührt. Proben für die OD₆₆₀- und Trockengewichtsbestimmung und HPLC-Analyse wurden in regelmäßigen Zeitintervallen entnommen. Die extrazellulären Metabolitenkonzentrationen, mit Ausnahme von Malat, wurden mittels einer Agilent-1260-HPLC bestimmt, die mit einer Bio-Rad HPX-87H-Säule ausgestattet war. Die Detektion erfolgte mittels eines Agilent-Brechungsindexdetektors und eines Agilent-1260-VWD-Detektors. Konzentrationen von Malat wurden unter Verwendung einer Bio-Rad HPX-87H-300-Säule (7,8 mm) bestimmt. Die Säule wurde mit Phosphorsäure (1,5 mM, 0,6 mL min⁻¹) eluiert. Die Detektion erfolgte mit einem Refraktometer (Waters 2414) und einem UV-Detektor (210 nm; Waters 484). Für Trockengewichtsmessungen wurden 5 ml Kultur über einen vorgewogenen Nitrozellulosefilter mit einer Porengröße von 0,45 µg filtriert. Anschließend wurden diese Filter mit Wasser gewaschen, 20 min bei 360 W in einem Mikrowellenofen getrocknet und erneut gewogen.

### Ergebnisse

Rekonstruktion des Stammes *UBR2*_{CBS}-DHA-SA-*An*DCT-02 Produktion von SA über einen reduktiven, redoxneutralen, CO₂-fixierenden und im Zytosol lokalisierten Stoffwechselweg.

Der in Xiberras et al. 2020 beschriebene konstruierte *S.-cerevisiae*-Stamm, der SA aus Glycerin und CO₂ produziert (Stamm "*UBR2*_{CBS}-DHA-SA-*An*DCT-02") basierte auf einem Stamm, der von Klein et al. (2016) konstruiert und veröffentlicht wurde. Es handelt sich dabei um ein Derivat des Stammes CEN.PK113-1A, welcher das sogenannte "DHA-Pathway-Modul II" trägt und von den mit diesem Modul versehenen Stämmen die höchste Wachstumsrate in synthetischem Glycerolmedium aufwies. Dieser Stamm wurde von Klein et al. (2016) als CEN.PK113-1A *UBR2_{CBS} gut1*Δ *Opgdh DAK1_{OE} CjFPS1 DAK1_{OE-2}* bezeichnet. Das sogenannte "DHA-Pathway-Modul II" nach Klein et al. (2016) enthielt eine Kassette zur heterologen Expression der NAD-abhängigen Glyceroldehydrogenase aus *Ogataea parapolymorpha* (*Opgdh*)*,* zwei Kassetten zur Überexpression der endogenen Dihydroxyacetonkinase (*DAK1*) sowie eine Expressionskassette für ein Aquaglyceroporin aus *Cyberlindnera jadinii* (*CjFPS1*) zur verbesserten Glycerolaufnahme. Um den Abbau von Glycerol über den nativen L-G3P-Weg zu unterbinden, wurde das gesamte Modul in den *GUT1*-Locus integriert, wodurch die Glycerolkinaseaktivität (die erste Reaktion des L-G3P-Wegs) eliminiert wurde. Eine weitere Modifikation in dem durch Klein et al. (2016) konstruierten Stamm bestand in dem Ersatz des endogenen UBR2-Allels durch das entsprechende Allel des natürlichen Isolats CBS 6412-13A. In Rahmen der vorliegenden Erfindung wurde ein Stamm konstruiert (hier kurz als *UBR2*_{CBS}-DHA bezeichnet), der dem von Klein et al. (2016) generierten Stamm CEN.PK113-1A *UBR2_{CBS} gut1*Δ *Opgdh DAK1_{OE} CjFPS1 DAK1_{OE-2}* prinzipiell ähnelt, sich jedoch in Bezug auf i) die verwendete Promotoren und Terminatoren und ii) die genomischen Integrationsorte der Expressionskassetten von diesem unterscheidet (vgl. Tabelle 1). Wie oben bereits erwähnt, wird in diesem Zusammenhang auch von einem "rekonstruierten Stamm" gesprochen, wobei der "rekonstruierte Stamm" mit dem ursprünglichen Stamm aus dem Stand der Technik nicht identisch ist. Der Ausgangsstamm und die Konstruktionsschritte, die für die Erzeugung des neuen "DHA-Pathway Stammes" *UBR2*_{CBS}-DHA unternommen wurden, sind oben detailliert beschrieben und unterscheiden sich von denen, die in Klein et al. (2016) ausgeführt sind. Im Detail wurde ein neu gestaltetes DHA-Pathway-Modul ("DHA-Modul") in das Genom integriert, wobei Erkenntnisse aus einer früheren Studie über die Stärke von *S.-cerevisiae*-Promotoren in Medium, das Glycerol als einzige Kohlenstoffquelle enthält, genutzt wurden (Ho et al., 2018). Im Vergleich zu dem zuvor erwähnten "DHA-Pathway-Modul II" wurde der Promotor P*_{PGK1},* der die Expression von *CjFPS1* kontrolliert, durch den stärkeren Promotor *P_{TDH3}* ersetzt. Zudem besteht das erfindungsgemäße DHA-Pathway-Modul nur aus einer einzigen (drei Expressionskassetten kombinierenden) Kassette für die Überexpression von *DAK1* (unter dem Promotor P*_{ADH2}*) (Ho et al., 2018). Das neu gestaltete DHA-Pathway-Modul, bestehend aus den drei Expressionskassetten für *Opgdh, DAK1* und *CjFPS1,* wurde am YGLCτ3-Locus integriert (Bai Flagfeldt et al., 2009), wie ebenfalls aus Tabelle 1 ersichtlich wird. Bai Flagfeldt et al. 2009 beschrieben mittlere bis hohe Expressionsniveaus, wenn die dort verwendete Reporter-Expressionskassette an dieser Stelle integriert wurde. Der *GUT1*-Locus, der von Xiberras et al. 2020 als Integrationsstelle verwendet wurde, ist bisher nicht bezüglich seines Einflusses auf die Expressionstärke charakterisiert worden. Um den L-G3P-Weg zu unterbinden, wurde *GUT1* in dem rekonstruierten Stamm *UBR2*_{CBS}-DHA unter Verwendung des Phleomycin-Resistenzmarkers deletiert.

Der rekonstruierte Stamm *UBR2*_{CBS}-DHA wurde dann zur Etablierung der SA-Produktion aus Glycerol unter Verwendung des gleichen Weges wie in Xiberras et al. (2020) beschrieben eingesetzt. Es wurden jedoch resynthetisierte Codierungssequenzen von *MDH3-R, RofumR, TbFRDg-R* und *AnDCT-02* verwendet, um die jeweiligen Expressionskassetten zu konstruieren. Die Codonoptimierung führte zu leicht unterschiedlichen DNA-Sequenzen, die jedoch keinen Einfluss auf die resultierende Aminosäuresequenz hatten. Bei der Auswahl der Promotor/Terminator-Kombinationen wurde berücksichtigt, dass eine homologe Rekombination im resultierenden Stamm zu verhindern ist, da eine solche zu einem Verlust von Expressionskassetten führen könnte. Das neu gestaltete SA-Modul und die neue Expressionskassette für *AnDCT-02* wurden alle am YPRCτ3-Locus integriert. Tabelle 1 (oben) zeigt einen Vergleich aller Modifikationen des Stamms *UBR2*_{CBS}-DHA-SA-*An*DCT-02, beschrieben in Xiberras et al. (2020), mit denen in der Hefezelle gemäß der vorliegenden Erfindung.

### Überexpression von PYC2 in UBR2_{CBS}-DHA-SA-AnDCT-02 gemäß Erfindung

Frühere Studien, die auf die SA-Produktion über den reduktiven, cytosolischen SA-Weg unter Verwendung von Glucose als Kohlenstoffquelle abzielten, überexprimierten auch die von *PYC2* codierte Pyruvatcarboxylase, um eine ausreichende Menge an Oxaloacetat, dem Vorläufer des etablierten SA-Erzeugungsweges, bereitzustellen (Yan et al. 2014;US 2015/0057425 A1). Auch Xiberras et al. (2020) überexprimierten *PYC2* in ihrem auf Glycerol wachsenden Stamm *UBR2*_{CBS}-DHA-SA-*An*DCT-02, stellten jedoch überraschend fest, dass dies einen negativen Einfluss auf den SA-Titer hatte und stattdessen vermehrt Ethanol gebildet wurde. Dennoch wurde in einer Ausführungsform der rekonstruierte (erfindungsgemäße) Stamm *UBR2*_{CBS}-DHA-SA-*An*DCT-02 auch mit einer Kassette zur Überexpression von *PYC2* ausgestattet. Die Kassette wurde zusammen mit dem SA-Modul am YPRCτ3-Locus integriert, wie oben beschrieben, und der Stamm wird hier als *UBR2*_{CBS}-DHA-SA-*An*DCT-02 *PYC2*ₒₑ bezeichnet.

### Die rekonstruierten Stämme zeigen eine stark verbesserte SA-Produktion

Die SA-Produktion des rekonstruierten (erfindungsgemäßen) Stammes *UBR2*_{CBS}-DHA-SA-*An*DCT-02 und der Variante, bei der *PYC2* überexprimiert wird, wurde in synthetischem Glycerolmedium in Schüttelkolben-Batch-Kultivierung unter den gleichen Bedingungen wie in Xiberras et al. (2020) beschrieben untersucht. Bemerkenswerterweise akkumulierten beide Stämme bis zu ~20 g/L SA im Kultivierungsmedium (Fig. 2A), was fast dem doppelten Titer entspricht, der mit dem in Xiberras et al. (2020) beschriebenen Stamm erreicht wurde. Der Basisstamm *UBR2*_{CBS}-DHA produzierte dagegen nur vernachlässigbare Mengen an SA, akkumulierte jedoch Ethanol (bis zu 3,5 g/L; Fig. 2D). Für die beiden erfindungsgemäßen SA-produzierenden Stämme wurde keine signifikante Ethanolproduktion festgestellt (Fig. 2D). Überraschend verkürzte die Überexpression von *PYC2* die Verzögerungsphase (Lag-Phase) des Stammes im Vergleich zu *UBR2*_{CBS}-DHA-SA-*An*DCT-02 und führte zu einer erheblich schnelleren Akkumulation von SA (Fig. 2A, 2C), was auf eine wesentlich höhere spezifische Glycerolverbrauchsrate in dem Stamm mit *PYC2*_{OE} zurückgeführt werden kann (Tabelle 6). Während der von Xiberras et al. (2020) beschriebene Stamm den maximalen SA-Titer nicht vor 168 Stunden Kultivierung erreichte (nicht dargestellt), erreichte der erfindungsgemäße Stamm *UBR2*_{CBS}-DHA-SA-*An*DCT-02 (ohne *PYC2*-Überexpression) das Maximum bereits nach 120 h, und der erfindungsgemäße *PYC2*-überexprimierende Stamm *UBR2_{CBS}*-DHA-SA-*An*DCT-02 *PYC2*ₒₑ bereits nach 72 h (s. Fig. 2A). Darüber hinaus hörten Wachstum, Glycerolverbrauch und SA-Produktion abrupt auf, sobald nur etwa 50% des zugeführten Glycerins verbraucht worden waren. Der mittlere pH-Wert war zu diesem Zeitpunkt unter 3,0 gefallen (nicht dargestellt). Wie in Tabelle 6 gezeigt, betrugen die maximalen Ausbeuten für *UBR2*_{CBS}-DHA-SA-*An*DCT-02 (gemäß Erfindung) und seines *PYC2* überexprimierenden Derivats 0,48 bzw. 0,53 ± 0,03 g/g.

**Tabelle 6. Maximale Titer und Ausbeuten bei der SA-Produktion aus Glycerol. Ein im Stand der Technik (Xiberras et al. 2020) beschriebener Stamm (UBR2_{CBS}-DHA-SA-AnDCT-02) wurde mit einem rekonstruierten erfindungsgemäßen Stamm UBR2_{CBS}-DHA-SA-AnDCT-02 und einer Variante davon (UBR2_{CBS}-DHA-SA-AnDCT02 PYC2ₒₑ) hinsichtlich der maximalen SA-Titer und -Ausbeuten in Schüttelkolben-Batch-Kultivierungen verglichen. Für die erfindungsgemäßen Stämme sind die spezifischen Glycerol-Abbauraten ebenfalls angegeben.**

| Stammbezeichnung | Max. SA-Titer [g L⁻¹] | Max. SA-Ausbeute [g SA / g Glycerol] | Spez. Glycerol-abbaurate [g g_{YDW}⁻¹ h⁻¹]^{∗} |
|---|---|---|---|
| *UBR2*_{CBS}-DHA-SA-AnDCT-02 (Xiberras et al. 2020) | 10,7 | 0,22 ±0,01 | |
| *UBR2*_{CBS}-DHA-SA-AnDCT-02 (Erfindung) | 20,55 ±0,50 | 0,48 ±0,01 | 0,17 ±0,00 |
| *UBR2*_{CBS}-DHA-SA-AnDCT-02 *PYC2*ₒₑ (Erfindung) | 19,46 ±0,50 | 0,53 ±0,01 | 0,26 ±0,00 |

| | | | |
|---|---|---|---|
| ^{∗} bezieht sich auf die spezifische Glycerol-Abbaurate zum Zeitpunkt des maximalen Glycerolverbrauchs | | | |

Der Glyoxylatzyklus trägt nicht zur SA-Produktion im erfindungsgemäßen Stamm *UBR2*_{CBS}-DHA-SA-*An*DCT-02 *PYC2*ₒₑ bei

In Xiberras et al. 2020 wurde bei der Herstellung von SA aus Glycerin festgestellt, dass die SA-Bildung bei dem dort beschrieben konstruierten Stamm stark vom Kohlenstofffluss über den PDH-Bypass sowie einen funktionsfähigen Glyoxylatzyklus abhängt (Xiberras et al., 2020). Tatsächlich schienen die Enzyme des konstruierten SA-Moduls und des nativen Glyoxylatzyklus auf synergistische Weise zu wirken, wie durch die Analyse von Stämmen gezeigt wurde, die jeweils nur einen der beiden Wege aufweisen. Die Aufhebung der SA-Produktion über den PDH-Bypass und den Glyoxylat-Zyklus wurde durch Deletion von *ICL1* erreicht, welches das Schlüsselenzym der Isocitrat-Lyase des Zyklus codiert (Xiberras et al., 2020). Die Beteiligung des Glyoxylatzyklus an der Herstellung von SA aus Glycerol zusätzlich zur Aktivität des reduktiven SA-Moduls ist jedoch unerwünscht, da dies nicht zu der geplanten redoxneutralen und CO₂-fixierenden Produktion von SA aus Glycerol führt. Zur Untersuchung, ob der oben erwähnte alternative Weg bei der Ausführungsform des erfindungsgemäßen Stamm mit einer starken Aktivität des SA-Moduls in Kombination mit einer Überexpression von *PYC2* noch zur Produktbildung beiträgt, wurde der Glyoxylatzyklus beim Stamm *UBR2*_{CBS}-DHA-SA-*An*DCT-02 *PYC2*ₒₑ durch Deletion von *ICL1* aufgehoben. Der *icl1Δ*-Stamm wurde parallel zu den oben beschriebenen Stämmen getestet. Es konnte festgestellt werden, dass die Deletion den Phänotyp des Stammes nicht verändert hat, was darauf hinweist, dass der alternative Weg über den PDH-Bypass und den Glyoxylatzyklus im Hintergrund des rekonstruierten erfindungsgemäßen Stammes keine signifikante Rolle spielen kann (Fig. 1, Tabelle 6).

### Die Zugabe von CaCO₃ zu Schüttelkolbenkulturen erhöht Titer und Ausbeute an produzierten Dicarbonsäuren wesentlich

Als nächstes wurde die Frage untersucht, ob die Verfügbarkeit von Bicarbonat, dem Co-Substrat der Pyruvat-Carboxylase-Reaktion, in den erfindungsgemäßen Stämmen mit dem optimierten rTCA-Weg geschwindigkeitsbestimmend geworden sein könnte. Um diese Hypothese zu testen, fügten wir den Schüttelkolbenkulturen CaCO₃ hinzu. Es wurde bereits gezeigt, dass die Zugabe von CaCO₃ zu Schüttelkolbenkulturen für die Produktion organischer Säuren das Kultivierungsmedium puffert (Yan et al., 2014; Zelle et al., 2010; Zelle et al., 2008) und gleichzeitig die Bikarbonatkonzentration im Medium erhöht (Zelle et al., 2010). Die Wirkung der Zugabe von CaCO₃ zum Kulturmedium wurde für die Stämme *UBR2*_{CBS}-DHA-SA-*An*DCT-02 und *UBR2*_{CBS}-DHA-SA-*An*DCT-02 *PYC2oe* sowie für den Stamm *UBR2*_{CBS}-DHA (nur ausgestattet mit dem DHA-Modul, ohne SA-Modul), der im Folgenden auch als Referenzstamm bezeichnet wird, in synthetischem Glycerol-Medium untersucht.

Zunächst ist anzumerken, dass die Anwesenheit von CaCO₃ tatsächlich eine Pufferwirkung zeigte, da der ermittelte pH-Wert der jeweiligen Kulturen relativ konstant blieb. Während der pH-Wert in den Kulturen der beiden SA-produzierenden Stämme während der Kultivierung zwischen 5 und 6 lag, schwankte der pH-Wert der Kultur mit dem Referenzstamm *UBR2*_{CBS}-DHA zwischen pH 6 und pH 7,5 (nicht dargestellt). In der ersten Phase der Kultivierung verhielten sich beide SA-produzierenden Stämme (mit und ohne Überexpression von *PYC2*) ähnlich im Vergleich zum Versuch ohne CaCO₃-Zugabe. Insbesondere der Stamm, der *PYC2* überexprimiert, reicherte SA schnell im Medium an. Diesmal hörten das Zellwachstum und die SA-Produktion jedoch nicht abrupt auf, sondern hielten an, bis nach 96 h Kultivierung ein maximaler SA-Titer von ~35 g L⁻¹ erreicht war (nicht dargestellt). Die höchste SA-Ausbeute (0,6 g g⁻¹ entsprechend 0,63 Cmol Cmol⁻¹) wurde nach 72 h beobachtet. Das Vorhandensein von CaCO₃ erlaubte allen Stämmen, signifikant mehr Glycerin zu verbrauchen. Es wurde praktisch kein Ethanol gebildet (nicht dargestellt). Bemerkenswerterweise wurden zusätzlich signifikante Mengen an Malat (MA) in das Medium sezerniert (nicht dargestellt). Die MA-Ausbeute nach 72 h (d. h. als die SA-Ausbeute am höchsten war) betrug etwa 0,1 Cmol Cmol⁻¹. Zu diesem Zeitpunkt landeten 53,2 % der im verbrauchten Glycerol verfügbaren Elektronen in Dicarbonsäuren (SA plus MA). Nach diesem Zeitpunkt nahmen die SA-Titer ab, während die MA-Titer gleichzeitig anstiegen, was auf eine SA-Aufnahme und teilweise Umwandlung in MA hinweist.

### Charakterisierung des Stammes UBR2_{CBS}-DHA-SA-AnDCT-02-PYC2oe in pHkontrollierten Bioreaktorexperimenten mit CO₂-Begasung und Abgasanalyse

Die Zugabe von CaCO₃ zu Schüttelkolbenkulturen könnte die Leistung der Stämme verbessert haben, indem der pH-Wert der Kultur gepuffert oder die Verfügbarkeit von Bicarbonat für die Pyruvat-Kinase-Reaktion erhöht wurde. Zur besseren Kontrolle dieser Kulturparameter und zur Quantifizierung der CO₂-Produktion/-Nutzung wurden Versuche mit dem Stamm *UBR2*_{CBS}-DHA-SA-AnDCT-02-PYC2oe in kontrollierten Bioreaktorkulturen durchgeführt. Da der entsprechende von Xiberras et al., 2020, beschriebene Stamm (ebenfalls bezeichnet als "*UBR2*_{CBS}-DHA-SA-AnDCT-02", jedoch mit anderer genetischer Ausstattung, s.o.) ebenfalls in Bioreaktoren charakterisiert wurde (s. Xiberras et al., 2020), wurden für die Versuche mit dem erfindungsgemäßen Stamm genau die gleichen experimentellen Bedingungen eingesetzt, um die Ergebnisse besser vergleichen zu können. Das synthetische Medium enthielt Ammoniumsulfat als Stickstoffquelle und 75,6 g L⁻¹ Glycerin, der pH wurde auf einem Wert von 5,0 gehalten und die Kulturen wurden mit CO₂-angereicherter Luft (~10 % (v/v) CO₂) mit einer Rate von 500 mL min⁻¹ begast. Unter diesen Bedingungen wurde ein maximaler SA-Titer von ~20 g L⁻¹ erhalten, mit einer Ausbeute von ca. 0,35 g g⁻¹ verbrauchtem Glycerin und einer maximalen spezifischen SA-Produktionsrate von 0,25 g g_{DCW}⁻¹ h⁻¹ (nicht dargestellt). Der Stamm reicherte unter diesen Bedingungen auch bis zu 9,0 g L⁻¹ Malat (0,11 g g⁻¹ verbrauchtes Glycerin) an (nicht dargestellt), was auch in Schüttelkolbenkulturen mit CaCO₃-Zusatz beobachtet wurde. Der Stamm produzierte im Bioreaktorexperiment auch kleine Mengen an Pyruvat und Citrat, jedoch waren die Konzentrationen dieser organischen Säuren (∼1,1 bzw. 0,6 g L⁻¹) wesentlich niedriger im Vergleich zu den Werten, die für den zuvor veröffentlichten SA-produzierenden Stamm (Xiberras et al., 2020) unter gleichen Bedingungen erhalten wurden.

Basierend auf der CO₂-Konzentration in der Gasmischung, die zur Begasung und zur kontinuierlichen Messung des Bioreaktorabgases verwendet wurde, ergab sich, dass in den produktiven ersten 71,75 h der Kultivierung ein Nettoverbrauch an CO₂ auftrat. Dies steht in deutlichem Gegensatz zum Verhalten des zuvor publizierten Stammes, bei dem zu dem entsprechenden Zeitpunkt (nach 71,5 h Kultivierung) ca. 32,7 % des verbrauchten Kohlenstoffs in gebildetem CO₂ gefunden wurden. Um die Verteilung des verbrauchten Kohlenstoffs in beiden Stammdesigns zu vergleichen, wurde im Falle des neuen Stammdesigns der Nettoverbrauch an CO₂ (entsprechend 5,7 ± 3,4 % des verbrauchten Kohlenstoffs) in die Kalkulation mit aufgenommen (Fig. 3.). Während ein ähnlicher Kohlenstoffanteil in der Biomassebildung landete, reichten die Stammmodifikationen in dieser Studie aus, um die CO₂-Bildung vollständig zu eliminieren, und führten zu einer wesentlich höheren Ausbeute an Bernsteinsäure sowie zu einer beachtlichen Nebenproduktbildung in Form von Malat ().

### Referenzen

Entian KD, Kötter P 2007, Yeast genetic strain and plasmid collections. Method Microbiol. 36: 629-666
Fakas, S., Makri, A., Bellou, S., Aggelis, G. 2009. Pathways to aerobic glycerol catabolism and their regulation. in: Microbial conversions of raw glycerol, (Ed.) G. Aggelis, Nova Science Publishers, Inc. New York, pp. 9-18.
Gancedo, C., Gancedo, J.M., Sols, A. 1968. Glycerol metabolism in yeasts. Pathways of utilization and production. Eur J Biochem, 5(2), 165-72.
Bai Flagfeldt, D., Siewers, V., Huang, L. and Nielsen, J. (2009), Characterization of chromosomal integration sites for heterologous gene expression in Saccharomyces cerevisiae. Yeast, 26: 545-551. https://doi.org/10.1002/yea.1705
Gonzalez, E., Fernandez, M.R., Larroy, C., Sola, L., Pericas, M.A., Pares, X., Biosca, J.A. 2000. Characterization of a (2R,3R)-2,3-butanediol dehydrogenase as the Saccharomyces cerevisiae YAL060W gene product. Disruption and induction of the gene. J Biol Chem, 275(46), 35876-85.
Ho, P.-W., Klein, M., Futschik, M., Nevoigt, E., Glycerol positive promoters for tailored metabolic engineering of the yeast Saccharomyces cerevisiae, FEMS Yeast Research, Volume 18, Issue 3, May 2018, foy019, https://doi.org/10.1093/femsyr/foy019
Ho, PW., Swinnen, S., Duitama, J. et al., 2017, The sole introduction of two single-point mutations establishes glycerol utilization in Saccharomyces cerevisiae CEN.PK derivatives, Biotechnol Biofuels 10, 10, doi:/10.1186/s13068-016-0696-6.
Hubmann, G, Guillouet, S., Nevoigt, E. 2011. Gpd1 and Gpd2 Fine-Tuning for Sustainable Reduction of Glycerol Formation in Saccharomyces cerevisiae. Appl. Env. Microbiol. 77, 5857-5867.
Itoh N, Tujibata Y, Liu JQ 1999. Cloning and overexpression in Escherichia coli of the gene encoding dihydroxyacetone kinase isoenzyme I from Schizosaccharomyces pombe, and its application to dihydroxyacetone phosphate production. Appl Microbiol Biotechnol. 51(2):193-200.
Izawa, S., Sato, M., Yokoigawa, K., Inoue, Y. 2004. Intracellular glycerol influences resistance to freeze stress in Saccharomyces cerevisiae: analysis of a quadruple mutant in glycerol dehydrogenase genes and glycerol-enriched cells. Appl Microbiol Biotechnol, 66(1), 108-14.
Jessop-Fabre MM, Jakociunas T, Stovicek V, Dai Z, Jensen MK, Keasling JD, Borodina I. Biotechnol J. 2016 Aug; 11(8): 1110-7, EasyClone-MarkerFree: A vector toolkit for marker-less integration of genes into Saccharomyces cerevisiae via CRISPR-Cas9. doi: 10.1002/biot.201600147.
Klein, M., Carrillo, M., Xiberras, J., Islam, Z., Swinnen, S., and Nevoigt, E., 2016, Towards the exploitation of glycerol's high reducing power in Saccharomyces cerevisiaebased bioprocesses. Metab. Eng. 38, 467-472.
Klein, M., Swinnen, S. Thevelein, J. Nevoigt, E. (2017). Glycerol metabolism and transport in yeast and fungi: Established knowledge and ambiguities. Environmental Microbiology. 19, 878-893, doi:10.1111/1462-2920.13617.
Klingenberg, M. 1970. Localization of the glycerol-phosphate dehydrogenase in the outer phase of the mitochondrial inner membrane. Eur J Biochem, 13(2), 247-52.
Liu, X., Mortensen, U.H., Workman, M. 2013. Expression and functional studies of genes involved in transport and metabolism of glycerol in Pachysolen tannophilus. Microb Cell Fact, 12, 27.
Los, A., den Dulk. A., Verwaal, R., et al. September 2015. Metabolic engineering of yeast for commercial production of succinic acid. Poster presented at the 32th International Specialized Symposium on Yeast, Perugia, Italien.
Mapelli, V. (Hrsg.) 2014: Yeast Metabolic Engineering, Methods and Protocols, Humana Press, ISBN 978-1-4939-0562-1
McKinlay, J.B., Vieille, C. and J.G. Zeikus. 2007. Prospects for a bio-based succinate industry. Appl Microbiol Biot 76:727 - 740.
Merico, A., Ragni, E., Galafassi, S., Popolo, L., Compagno, C. 2011. Generation of an evolved Saccharomyces cerevisiae strain with a high freeze tolerance and an improved ability to grow on glycerol. J Ind Microbiol Biotechnol, 38(8), 1037-44.
Nevoigt, E. 2008. Progress in metabolic engineering of Saccharomyces cerevisiae. Microbiol Mol Biol Rev, 72(3), 379-412.
Nevoigt, E., Kohnke, J., Fischer, CR., Alper, H., Stahl, U., Stephanopoulos, G. 2006. Engineering of promoter replacement cassettes for fine-tuning of gene expression in Saccharomyces cerevisiae. Appl. Environ. Microbiol. 72: 5266-5273
Nijkamp, J.F., van den Broek, M., Datema, E. et al., 2012, De novo sequencing, assembly and analysis of the genome of the laboratory strain Saccharomyces cerevisiae CEN.PK113-7D, a model for modern industrial biotechnology, Microb Cell Fact 11, 36, doi:10.1186/1475-2859-11-36.
Ochoa-Estopier A, Lesage J, Gorret N, Guillouet SE, 2011, Kinetic analysis of a Saccharomyces cerevisiae strain adapted for improved growth on glycerol: implications for the development of yeast bioprocesses on glycerol. Bioresour Technol. 2011;102:1521-7. Otero, J. M., Cimini, D., Patil, K. R., et al. 2013. Industrial Systems Biology of Saccharomyces cerevisiae Enables Novel Succinic Acid Cell Factory. PLoS ONE 8(1):e54144.
Raab, A.M., Gebhardt, G., Bolotina, N., et al. 2010. Metabolic engineering of Saccharomyces cerevisiae for the biotechnological production of succinic acid. Metabolic Engineering 12(6):518-25.
Sprague, G.F., Cronan, J.E. 1977. Isolation and characterization of Saccharomyces cerevisiae mutants defective in glycerol catabolism. J Bacteriol, 129(3), 1335-42. Swinnen, S., Klein, M., Carrillo, M., McInnes, J., Nguyen, H.T., Nevoigt, E. 2013. Reevaluation of glycerol utilization in Saccharomyces cerevisiae: characterization of an isolate that grows on glycerol without supporting supplements. Biotechnol Biofuels, 6(1), 157.
Swinnen S, Ho PW, Klein M, Nevoigt E. Genetic determinants for enhanced glycerol growth of Saccharomyces cerevisiae. Metab Eng. 2016 Jul;36:68-79. doi: 10.1016/j.ymben.2016.03.003. Epub 2016 Mar 10. PMID: 26971668.
Van Maris AJ, Geertman JM, Vermeulen A, Groothuizen MK, Winkler AA, Piper MD, van Dijken JP, Pronk JT., 2004, Directed evolution of pyruvate decarboxylase-negative Saccharomyces cerevisiae, yielding a C2-independent, glucose-tolerant, and pyruvatehyperproducing yeast. Appl Environ Microbiol. 2004 Jan;70(1): 159-66.
van Dijken JP, Bauer J, Brambilla L, Duboc P, Francois JM, Gancedo C, Giuseppin ML, Heijnen JJ, Hoare M, Lange HC, et al, 2000, An interlaboratory comparison of physiological and genetic properties of four Saccharomyces cerevisiae strains, Enzyme Microb Tech. 26: 706-714, doi: 10.1016/S0141-0229(00)00162-9.
Xiberras J, Klein M, de Hulster E, Mans R, Nevoigt E. Engineering Saccharomyces cerevisiae for Succinic Acid Production From Glycerol and Carbon Dioxide. Front Bioeng Biotechnol. 2020 Jun 26;8:566. doi: 10.3389/fbioe.2020.00566. PMID: 32671027; PMCID: PMC7332542.
Yan, D., Wang, C., Zhou, J., et al. (2014) Construction of reductive pathway in Saccharomyces cerevisiae for effective succinic acid fermentation at low pH value. Bioresource Technology 156: 232-239.
Zelle, R. M., Trueheart, J., Harrison, J. C., et al. 2010. Phosphoenolpyruvate carboxykinase as the sole anaplerotic enzyme in Saccharomyces cerevisiae. Applied and Environmental Microbiology 76:5383-5389.
Zelle, R. M., de Hulster, E., van Winden, W. A., de Waard, P., Dijkema, C., Winkler, A. A., Geertman, J. M., van Dijken, J. P., Pronk, J. T., van Maris, A. J., 2008. Malic acid production by Saccharomyces cerevisiae: engineering of pyruvate carboxylation, oxaloacetate reduction, and malate export. Appl Environ Microbiol. 74, 2766-77.

## Patentansprüche

1. Gentechnisch veränderte Hefezelle der Gattung *Saccharomyces* zur biotechnologischen Herstellung von Bernsteinsäure aus Glycerol, wobei die Hefezelle dahingehend gentechnisch verändert ist, dass
a) der Abbau von Glycerol zu Dihydroxyacetonphosphat über den Glycerol-3-Phosphat-Weg blockiert ist, und
b) die folgenden Gene in den LTR-Insertionslokus YPRCτ3 exprimierbar inseriert sind:
i) *ScMDH3-R* unter Kontrolle des Promotors **P***_{JEN1}* und funktional verbunden mit der Terminationssequenz **T***_{IDP1},*
ii) *RofumR* unter Kontrolles des Promotors **P***_{HOR7}* und funktional verbunden mit der Terminationssequenz **T***_{DIT1},*
iii) *TbFRDg-R* unter Kontrolle des Promotors **P***_{FBA1}* und funktional verbunden mit der Terminationssequenz **T***_{ADH1},*
iv) *AnDCT02* unter Kontrolle des Promotors **P***_{COX7}* und funktional verbunden mit der Terminationssequenz **T***_{CYC1}**,*** und
c) die folgenden Gene in den LTR-Insertionslokus YGLCτ3 exprimierbar inseriert sind:
i) *CjFPS1* unter Kontrolle des Promotors **P***_{TDH3}* und funktional verbunden mit der Terminationssequenz **T***_{RPL15A,}*
ii) *Opgdh* unter Kontrolles des Promotors **P***_{TEF1}* und funktional verbunden mit der Terminationssequenz **T***_{CYC1},* und
iii) *ScDAK1* unter Kontrolle des Promotors **P***_{ADH2}* und funktional verbunden mit der Terminationssequenz **T***_{TPS1}.*

2. Gentechnisch veränderte Hefezelle nach Anspruch 1, wobei die Hefezelle ferner dahingehend gentechnisch verändert ist, dass
d) das Allel *UBR2* gegen das Allel *UBR2_{CBS 6412-13A},* das Allel *UBR2_{CEN.PK113}-_{7DJL1},* das Allel *UBR2_{CEN.PK113-1A PW-1}* oder das Allel *UBR2_{CEN.PK113-1A PW-2}* ausgetauscht ist.

3. Gentechnisch veränderte Hefezelle nach einem der Ansprüche 1 oder 2, wobei der Abbau von Glycerol zu Dihydroxyacetonphosphat über den Glycerol-3-Phosphat-Weg durch Deletion des für die homologe Glycerolkinase kodierenden Gens *GUT1* blockiert ist.

4. Gentechnisch veränderte Hefezelle nach einem der vorhergehenden Ansprüche, wobei es sich um eine Hefezelle handelt, die nach dem Budapester Vertrag unter der Zugriffsnummer 58096 bei der belgisch koordinierten Sammlung von Mikroorganismen, BCCM, hinterlegt ist.

5. Gentechnisch veränderte Hefezelle nach einem der vorhergehenden Ansprüche, wobei die Hefezelle zusätzlich dahingehend gentechnisch verändert ist, dass die Pyruvatcarboxylase 2, Pyc2, überexprimiert wird.

6. Gentechnisch veränderte Hefezelle nach einem der vorhergehenden Ansprüche, wobei die Hefezelle eine Hefezelle der Art *Saccharomyces cerevisiae* ist.

7. Verfahren zur biotechnologischen Herstellung von Bernsteinsäure aus Glycerol, wobei eine gentechnisch veränderte Hefezelle nach einem der vorhergehenden Ansprüche in einem glycerolhaltigen Kulturmedium unter geeigneten Bedingungen kultiviert und Bernsteinsäure von dem Kulturmedium abgetrennt wird.

8. Verfahren nach Anspruch 7, wobei die biotechnologische Herstellung von Bernsteinsäure fermentativ erfolgt.

9. Verfahren nach Anspruch 7 oder 8, wobei Glycerol einzige Kohlenstoffquelle ist.

10. Verfahren nach einem der Ansprüche 7 bis 9, wobei die gentechnisch veränderte Hefezelle in dem glycerolhaltigen Kulturmedium in Gegenwart von dem Kulturmedium zugegebenem CaCO₃ oder unter Zufuhr von gasförmigem Kohlendioxid in das Kulturmedium kultiviert wird.

11. Verfahren nach Anspruch 10, wobei die gentechnisch veränderte Hefezelle eine Hefezelle nach einem der Ansprüch 5 oder 6 ist.
